# EUROPÄISCHE PATENTSCHRIFT

(11) **EP 0 567 472 B1**
(45) Veröffentlichungstag und Bekanntmachung des Hinweises auf die Patenterteilung: **07.10.1998**
(21) Anmeldenummer: 92901412.4
(22) Anmeldetag: 21.12.1991
(51) Int. Cl.: C07J 63/00, A61K 31/565, A61K 31/58

(54) **D-HOMO-(16-EN)-11 BETA-ARYL-4-ESTRENE,VERFAHREN ZU IHRER HERSTELLUNG SOWIE DEREN VERWENDUNG ALS ARZNEIMITTEL.**
D-HOMO-(16-EN)-11 BETA-ARYL-4-ESTRENES, PROCESS FOR PRODUCING THEM AND THEIR USE AS MEDICAMENTS
D-HOMO-(16-ENE)-11 BETA-ARYL-4-ESTRENES, LEUR PROCEDE DE PRODUCTION ET LEUR UTILISATION COMME MEDICAMENTS.

(30) Priorität: 22.12.1990 DE 4042005
(43) Veröffentlichungstag der Anmeldung: 03.11.1993
(73) Patentinhaber: SCHERING AKTIENGESELLSCHAFT, 13342 Berlin (DE)
(72) Erfinder: SCHWEDE, Wolfgang, D-1000 Berlin 10 (DE); OTTOW, Eckhard, D-1000 Berlin 45 (DE); CLEVE, Arwed, D-1000 Berlin 42 (DE); ELGER, Walter, D-1000 Berlin 33 (DE); CHWALISZ, Krzysztof, D-1000 Berlin 45 (DE); SCHNEIDER, Martin, D-1000 Berlin 28 (DE)
(86) Internationale Anmeldenummer: EP9102495
(87) Internationale Veröffentlichungsnummer: WO9211279

(56) Entgegenhaltungen:
- EP-A- 0 116 974
- EP-A- 0 127 864
- EP-A- 0 147 361

## Beschreibung

Die vorliegende Erfindung betrifft Verbindungen der allgemeinen Formel I 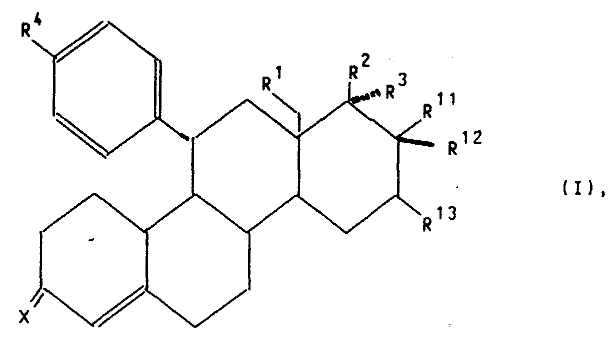 worin
- X: für ein Sauerstoffatom, die Hydroxyiminogruppierung >N∼OH oder zwei Wasserstoffatome,
- R¹: für ein Wasserstoffatom oder eine Methylgruppe,
- R²: für eine Hydroxygruppe, eine C₁-C₁₀-Alkoxy- oder C₁-C₁₀-Acyloxygruppe,
- R³: für ein Wasserstoffatom, die Gruppierung -(CH₂)ₙCH₂Z, wobei n 0, 1, 2, 3, 4 oder 5 ist, Z ein Wasserstoffatom, die Cyanogruppe oder den Rest-OR⁵ mit R⁵=H, C₁-C₁₀-Alkyl oder C₁-C₁₀-Acyl bedeuten, die Gruppierung -(CH₂)ₘC≡C-Y, wobei m 0, 1 oder 2 und Y ein Wasserstoff-, Fluor-, Chlor-Brom-oder Jod-Atom, einen C₁-C₁₀-Hydroxyalkyl-, C₁-C₁₀-Alkoxyalkyl-, C₁-C₁₀-Acyloxyalkylrest, oder wenn m = 0 ist, zusätzlich eine Methylgruppe bedeuten, die Gruppierung -(CH₂)ₚ-CH=CH-(CH₂)ₖCH₂R⁶, wobei p 0 oder 1 und k 0, 1 oder 2 und R⁶ ein Wasserstoffatom, eine Hydroxygruppe, einen C₁-C₄-Alkoxy- oder C₁-C₄-Acyloxyrest bedeuten,
oder aber R² und R³ gemeinsam für einen Rest der Formel 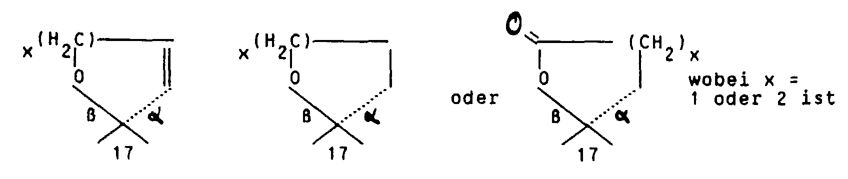
R⁴ für ein Wasserstoffatom, eine Cyanogruppe, ein Chlor-, Fluor-, Brom-, Jodatom, für eine Trialkylsilyl-, Trialkylstannylgruppe, für einen geradkettigen oder verzweigten, gesättigten oder ungesättigten C₁-C₈-Alkyl-, -Acyl-oder Alkoxyalkylrest, für eine 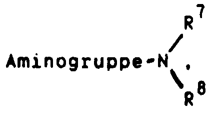 in welcher R⁷ und R⁸ unabhängig voneinander ein Wasserstoffatom oder eine C₁-C₄-Alkylgruppe bedeuten, oder für ein entsprechendes Aminoxid 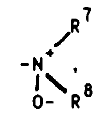 oder für die Gruppierungen -OR⁹ oder -S(O)ᵢR⁹ mit i = 0, 1 oder 2, in welchen R⁹ ein Wasserstoffatom, eine Methyl-, Ethyl-, Propyl-, Isopropyl-, Methoxyphenyl-, Allyl-oder eine 2-Dimethylaminoethylgruppe bedeuten, oder für einen Heteroarylrest der Formel Iα 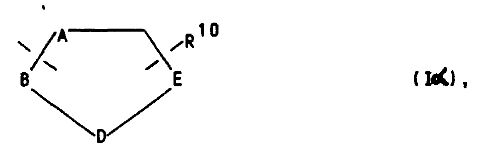 in welchem A ein Stickstoff-, Sauerstoff- oder Schwefelatom, -B-D-E-die Elementenfolge -C-C-C-, -N-C-C- oder -C-N-C- und R¹⁰ ein Wasserstoffatom, eine Cyanogruppe, ein Chlor-, Fluor-, Brom-, Jodatom, eine Trialkylsilyl-, Trialkylstannylgruppe, einen geradkettigen oder verzweigten, gesättigten oder ungesättigten C₁-C₈-Alkyl-, -Acyl-oder Alkoxyalkylrest, für eine 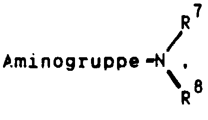 in welcher R⁷ und R⁸ unabhängig voneinander ein Wasserstoffatom oder eine C₁-C₄-Alkylgruppe bedeuten, oder ein entsprechendes Aminoxid 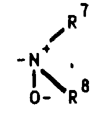 oder die Gruppierung -OR⁹ oder -S(O)ᵢR⁹ mit i = 0, 1 oder 2, in welchen R⁹ ein Wasserstoffatom, eine Methyl-, Ethyl-, Propyl-, Isopropyl-, Methoxyphenyl-, Allyl-oder eine 2-Dimethylaminoethylgruppe bedeuten, symbolisieren,
oder für einen Heteroarylrest der Formel Iβ 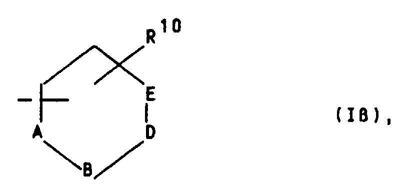 in welchem A ein Stickstoffatom und -B-D-E- die Elementenfolge -C-C-C-, -N-C-C-, -C-N-C- oder -C-C-N- bedeuten un R¹⁰ die bereits angegebene Bedeutung hat,
oder für einen Phenylrest der Formel Iγ 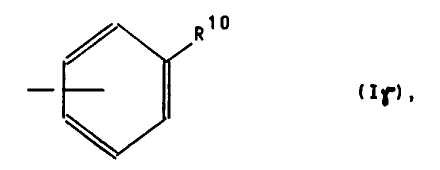 worin R¹⁰ die bereits angegebene Bedeutung hat,
R¹¹ für ein Fluor-, Chlor- oder Bromatom, wobei dann R¹² und R¹³ gemeinsam eine zusätzliche Bindung bedeuten oder
R¹¹ für einen gerad- oder verzweigtkettigen C₁-C₄-Alkylrest oder ein Wasserstoffatom, wobei dann R¹² und R¹³ je ein Wasserstoffatom oder gemeinsam eine zusätzliche Bindung bedeuten,
stehen,
sowie deren pharmakologisch verträgliche Additionssalze mit Säuren, Verfahren zu ihrer Herstellung, diese Verbindungen enthaltende pharmazeutische Präparate, ihre Verwendung zur Herstellung von Arzneimitteln sowie die dazu benötigten neuen Zwischenprodukte.

Die Erfindung betrifft insbesondere Verbindungen, in denen X für ein Sauerstoffatom steht.

Die in R², R³, R⁵ und Y der allgemeinen Formel I enthaltenen Alkoxy-, Acyloxy-, Alkyl-, Acyl- sowie Hydroxyalkylgruppen sollen jeweils 1 bis 10 und die Alkoxyalkyl- oder Acyloxyalkylgruppen in Y 2 bis 10 Kohlenstoffatome enthalten. Dabei sind als bevorzugte Gruppen von den Alkoxygruppen die Methoxy-, Ethoxy-, Propoxy- und Isopropoxygruppe zu nennen, von den Acyl(oxy)gruppen kommt der Formyl(oxy)-, Acetyl(oxy)- und Propionyl(oxy)-gruppe besondere Bedeutung zu.

Bei den Alkylgruppen sind vor allem die Methyl-, Ethyl-, Propyl-, Isopropyl-sowie tert.-Butylgruppe zu nennen und von den Hydroxyalkylgruppen sind die entsprechenden, in beliebiger Stellung mit einer Hydroxygruppe substituierten Reste bevorzugt.

Für n kommt insbesondere 0, 1, 2 und 3 infrage; wenn Z=CN, ist eine Cyanomethyl gruppe (n=0) besonders bevorzugt. Außer den bereits genannten Gruppen kann Y vorzugsweise auch ein Wasserstoff-, Chlor- oder Brom-Atom sein.

Von den Alkenylresten in R³ sind die Propenyl- und Butenylgruppen, die in der E- oder Z-Konfigurationm vorliegen können, bevorzugt, das heißt, wenn R³ für -(CH₂)ₚ-CH=CH-(CH₂)ₖ-CH₂-R⁶ steht, dann soll k vorzugsweise 0 oder 1 und p=0 sein.
Unter den für R⁶ genannten Alkoxy- oder Acyloxygruppen, die sowohl geradkettig als auch verzweigt sein können, sind die Methoxy-, Ethoxy-, Propoxy-, Isopropoxy- bzw. die Formyloxy-, Acetyloxy- und Propionyloxygruppe besonders bevorzugt.

Von den C₁-C₈-Alkyl- und Alkoxyalkylresten, die für R⁴ stehen können, sind dies vor allem der Methyl-, Ethyl-, Propyl-, Isopropyl-, Cyclopentyl- und Cyclohexylrest bzw. die Alkoxymethyl- bzw. 1- oder 2-Alkoxyethylgruppen mit den genannten Alkylresten; von den C₁-C₈-Acylresten für R⁴ ist insbesondere an den Acetyl-, Propionyl- und Isobutylrylrest gedacht Steht R⁴ für die 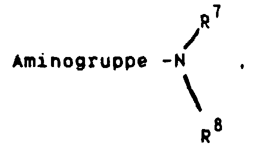 bedeuten R⁷ und R⁸ vorzugsweise jeweils einen Methylrest, doch auch dem Ethylrest kommt besondere Bedeutung zu, wobei dann entweder beide Reste am Stickstoffatom für einen Ethylrest oder einer für einen Methyl- und einer für einen Ethylrest stehen. Für den Substituenten R⁹ sind die Methyl-, Ethyl- und 2-(Dimethylamino)ethylgruppe besonders hervorzuheben.

Von den gemäß Formel Iα möglichen Heteroarylresten sind der 3-Thienyl-, 3-Furyl- und 3-Pyrrolylrest bevorzugt mit R¹⁰ in der Bedeutung einer Cyano-, Methoxy- oder Dimethylaminogruppe.

Als Heteroarylreste der Formel Iβ kommen erfindungsgemäß insbesondere der 3-oder 4-Pyridyl-, der 5-Pyrimidinyl-, 4-Pyridazinyl- oder Pyrazinylrest infrage.

Der Phenylrest der Formel Iγ weist als Substituenten R¹⁰ insbesondere die Cyano-, Methoxy- oder Dimethylaminogruppe auf, wobei sich wiederum diese Substituenten bevorzugt in der p-Position des Phenylringes befinden.

Nachstehend genannte Verbindungen sind erfindungsgemäß besonders bevorzugt:
11β-Aryl-D-homo-4,9-estradien- und -4,9,16-estratrien-3-one mit einer 17β-Hydroxy- und 17α-(3-Hydroxypropyl)gruppe, mit einer 17-Spironolakton-gruppe, mit einem Oxathiolan-S-oxid-, Oxazolidin- oder Oxathiazolidin-S-oxid-Ring (unter Einschluß des 17-Kohlenstoffatoms) gehen aus der EP-A-0 116 974,
11β-Aryl-D-homo-4,9,16-estratrien-3-one mit 17β-Hydroxy- und einem 17α-Ethinyl-, -Chlorethinyl- oder -Propinylsubstituenten gehen aus der EP-A-0 127 864 sowie 11β-Aryl-D-homo-4,9-estradien- und -4,9,16-estratrien-3-one mit 17β-Hydroxy- und 17α-(3-Hydroxyprop-1(Z)enyl)- oder 17α-(3-Acyloxyprop-1(Z)enyl)- Substituenten gehen aus der EP-A-0 147 361 hervor. Die bekannten Verbindungen sind alle als solche mit antigestagener Wirksamkeit beschrieben.
17aβ-Hydroxy-11β-(4-methoxyphenyl)-17aα-(1-propinyl)-17a-homoestra-4,16-dien-3-on
17aβ-Hydroxy-11β-(4-methoxyphenyl)-17aα-methyl-17α-homoestra-4,16-dien-3-on
11β-[4-(3-Furanyl)phenyl]-17aβ-hydroxy-17aα-(1-propinyl)-17a-homoestra-4,16-dien-3-on
11β-(4-Acetylphenyl)-17aβ-hydroxy-17aα-(1-propinyl)-17a-homoestra-4,16-dien-3-on
17aβ-Hydroxy-17aα-(3-hydroxypropyl)-11β-[4-(3-pyridinyl)phenyl]-17a-homoestra-4,16-dien-3-on
(Z)-4'-[17aβ-Hydroxy-17aα-(3-hydroxy-1-propenyl)-3-oxo-17a-homo-4,16-dien-11β-yl]-[1,1'-biphenyl]-4-carbonitril
11β-[4-(3-Furanyl)phenyl]-17aβ-hydroxy-17aα-methyl-17a-homoestra-4,16-dien-3-on
11β-(4-Acetylphenyl)-17aβ-hydroxy-17aα-(3-hydroxypropyl)-17a-homoestra-4-en-3-on
11β-(4-Acetylphenyl)-4',5'-dihydrospiro[17a-homoestra-4-en-17aβ,2'(3H)-furan]-3-on
(Z)-4'-[17aβ-Hydroxy-17aα-(3-hydroxy-1-propenyl)-3-oxo-17a-homoestr-4-en-11β-yl][1,1'-biphenyl]-4-carbonitril
17-Chlor-11β-(4-methoxyphenyl)-17aβ-hydroxy-17aα-(1-propinyl)-17a-homoestra-4,16-dien-3-on
17-Chlor-17aβ-hydroxy-17aα-(1-propinyl)-11β-[4-(3-pyridinyl)phenyl]-17a-homoestra-4,16-dien-3-on
11β-(4-Acetylphenyl)-17-chlor-17aβ-hydroxy-17aα-(1-propinyl)-17a-homoestra-4,16-dien-3-on
17-Chlor-11β-[4-(3-furanyl)phenyl]-17aβ-hydroxy-17aα-(1-propinyl)-17a-homoestra-4,16-dien-3-on
17-Chlor-11β-[4-(3-furanyl)phenyl]-17aβ-hydroxy-17aα-methyl-17a-homoestra-4,16-dien-3-on
4'-[17-Chlor-17aβ-hydroxy-17aα-methyl-3-oxo-17a-homoestra-4,16-dien-11β-yl][1,1'-biphenyl]4-carbonitril
(Z)-11β-(4-Acetylphenyl)-17-chlor-17aβ-hydroxy-17aα-(3-hydroxy-1-propenyl)-17a-homoestra-4,16-dien-3-on
11β-(4-Acetylphenyl)-17-chlor-17aβ-hydroxy-17aα-(3-hydroxypropyl)-17a-homoestra-4,16-dien-3-on
17-Chlor-11β-[4-(3-furanyl)phenyl]-17aβ-hydroxy-3-oxo-17a-homoestra-4,16-dien-17aα-acetonitril
11β-(4-Acetylphenyl)-17-fluor-17aβ-hydroxy-17aα-methyl-17a-homoestra-4,16-dien-3-on
11β-(4-Acetylphenyl)-17-fluor-17aβ-hydroxy-17aα-(3-hydroxypropyl)-17a-homoestra-4,16-dien-3-on
11β-(4-Acetylphenyl)-17-fluor-17aβ-hydroxy-17aα-(1-propinyl)-17a-homoestra-4,16-dien-3-on
17aβ-Hydroxy-11β-(4-methoxyphenyl)-17aα-(1-propinyl)-17a-homoestr-4-en-3-on

Die Herstellung der erfindungsgemäßen Verbindungen erfolgt gemäß nachfolgendem Reaktionsschema: 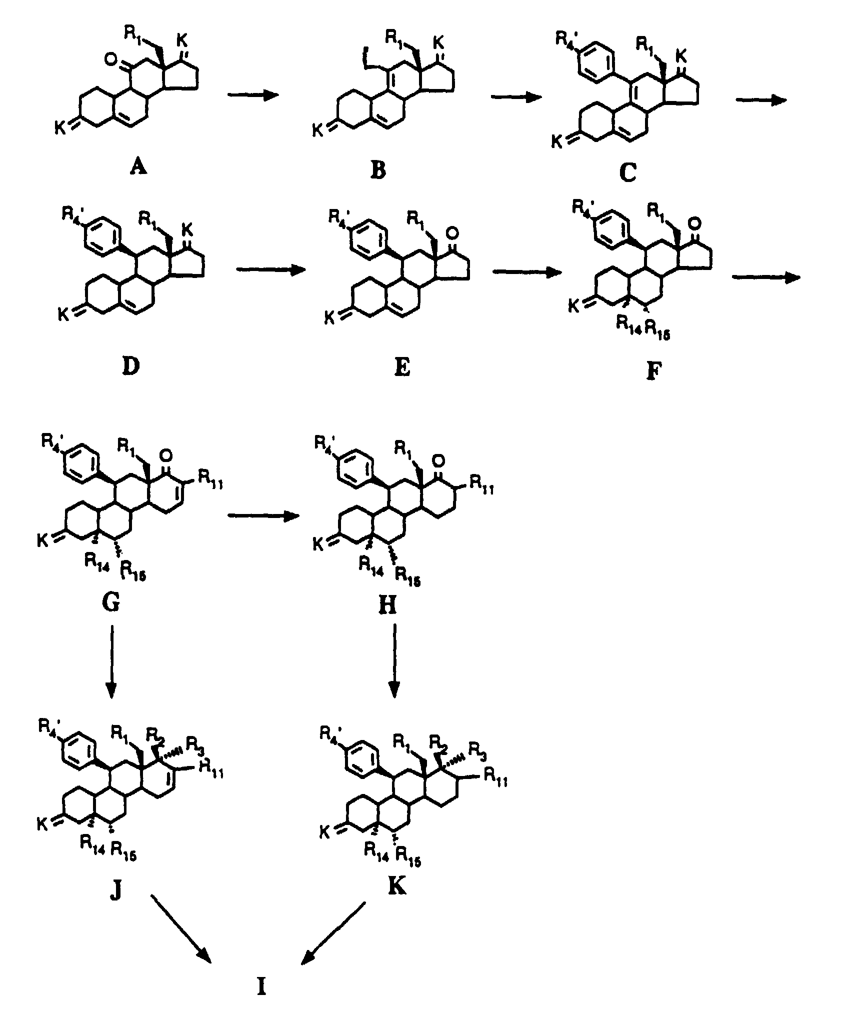 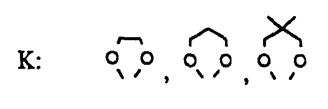 oder andere Sauerstoffketale Gemäß der vorliegenden Erfindung wird Verbindung A (Recl. Trav. Chim. Bays-Pas 107, 331, (1988)) zunächst in eine Verbindung der Formel B überführt, wobei L für eine Perfluoralkylsulfonyloxygruppe CₙF₂ₙ₊₁SO₂O-(n= 1, 2, 3, 4) steht.

Verbindung B wird in Gegenwart einer katalytischen Menge eines Übergangsmetallkatalysators mit einer Arylverbindung der allgemeinen Formel Z 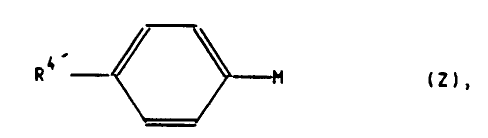 worin M für einen der Reste
-B(Alkyl)₂
-Sn(Alkyl)₃ Alkyl C₁-C₄-Alkylrest
-B(OH)2
-ZnHal
-MgHal Hal = Cl, Br, J
und R^{4'} für einen der unter R⁴ genannten Reste stehen,
zu einer Verbindung der allgemeinen Formel C,
worin R⁴ die in Formel I und R^{4'} die in Formel Z angegebene Bedeutung haben und gegebenenfalls, wenn R⁴ in der Formel I eine andere Bedeutung als R^{4'} in der Formel C haben soll, eine Verbindung der allgemeinen Formel C, worin R^{4'} für ein Bromatom steht oder nach Überführung einer für R^{4'} stehenden Methoxygruppe in eine Perfluoralkylsulfonyloxygruppe CₙF₂ₙ₊₁SO₂O-(n=1,2,3,4), mit einer Verbindung der allgemeinen Formel VI

R⁴-M (VI),

worin R⁴ die letztlich für diesen Substituenten in der Formel I gewünschte und M die bereits in Formel Z angegebene Bedeutung haben, umgesetzt.

Für L in der Verbindung B steht vorzugsweise die Trifluormethylsulfonyloxygruppe. Als Übergangsmetallkatalysator zur Kupplung der Arylverbindung der allgemeinen Formel Z mit der die Abgangsgruppe L aufweisenden Verbindung dient gemäß den Beispielen vorliegender Erfindung Palladiumtetrakistriphenylphosphin (siehe nachstehend angegebene Literatur); genausogut könnte aber Nickeltetrakistriphenylphosphin oder ähnliche Übergangsmetallkatalysatoren verwendet werden.

Die Variante, daß der letztendlich gewünschte Substituent R⁴ über die Funktionalisierung eines Brom- oder Methoxysubstituenten R^{4'} in der Verbindung C eingeführt wird, ist dann zu wählen, wenn die zu kuppelnde Arylverbindung der allgemeinen Formel Z, worin R^{4'} bereits mit R⁴ identisch ist, nicht zugänglich oder zur Kupplung nicht geeignet ist. Übergangsmetallkatalysierte Arylkupplungsreaktionen von Verbindungen des Typs der allgemeinen Formel Z mit Verbindungen, die eine Abgangsgruppe tragen, sind beispielsweise beschrieben in: mit -Sn(Alkyl)₃-substituierten Aromaten: J.E. McMurry and S. Mohanraj, Tetrahydron Letters, 24, No. 27, S. 2723-2726, 1983; X. Lu und J. Zhu, Communications, S. 726-727, 1987; Q.-Y. Chen und Z.-Y. Yang, Tetrahedron Letters 27, No. 10, S. 1171-1174, 1986; S. Cacchi, P.G. Ciattini, E. Morera und G. Ortar, Tetrahedron Letters 27, No. 33, S. 3931-3934, 1986; A.H. Echavarren und J.K. Stille, J.Am.Chem. Soc. 1987, 109, S. 5478-5486 und J.Am.Chem.Soc. 1988, 110, S. 1557: mit -B(OH)₂ und -B(OAlkyl)₂-substituierten Aromaten: Y. Hoshino, N. Hiyaura und A. Suzuki, Bull.Chem.Soc. Jpn. 61, 3008 (1988): H. Hatsubasa, K. Seto, T. Tahara and S. Takahashi; Bull.Chem. Soc. Jpn, 62, 3896 (1989); mit -ZnCl-substituierten Aromaten: R. McCague, Tet. Lett., 28, 701 (1987); A. Arcadi, A. Burini, S. Cacchi, M. Delmastro, F. Harinelli, B. Pietroni, Syn. Les., 1, 1990, S. 47.

Die Verbindungen der allgemeinen Formel D, die als Ausgangsprodukte für die Herstellung der 10β-H-Steroide der allgemeinen Formel I geeignet sind, lassen sich bequem herstellen, indem eine Verbindung der Formel C, worin R⁴ und R¹ die in den Formeln angegebene Bedeutung haben, ohne Zerstörung des aromatischen Systems und der 5,6-Doppelbindung zu einer Verbindung der allgemeinen Formel D, worin R⁴ und R¹ die bereits angegebene Bedeutung haben, reduziert wird.

Bei der Reduktion von C bildet sich die 11β-Arylverbindung D (stereoselektive Reduktion).
Zur Reduktion der 9(11)-Doppelbindung in C kommen erfindungsgemäß verschiedene Methoden infrage:
erfindungsgemäß bevorzugt ist die Reduktion mit einem elektropositiven Metall in einem elektronensolvatisierenden Lösungsmittel oder in einem einen Lösungsvermittler enthaltenden Lösungsmittel. Als elektronensolvatisierendes Lösungsmittel kommt in erster Linie Ammoniak inbetracht.

Für die Reduktion genügen bereits equimolare Mengen Reduktionsmittel, es kann jedoch auch ein beträchtlicher Überschuß Reduktionsmittel verwendet werden, ohne daß das aromatische System und/oder die 5,6-Doppelbindung angegriffen werden/wird.
Als elektropositive Metalle sind alle für eine Birch-Reduktion geeigneten Metalle verwendbar. Erfindungsgemäß sind Lithium, Kalium, Natrium und Calcium -und von diesen Lithium insbesondere - bevorzugt.

Selektive Spaltung der Ketoschutzgruppe in 17-Position mit einer schwachen Säure (Essigsäure, Oxalsäure) ergibt die Verbindung E.

Die Verbindungen der Formel E werden epoxidiert, zum Beispiel unter Verwendung organischer Persäure oder mit Wasserstoffperoxid in Gegenwart von Hexachloraceton oder Nitrotrifluoracetophenon. Die folgenden Umsetzungen werden entweder in Gegenwart des Epoxids oder aber durch reduktive Öffnung unter Verwendung komplexer Hydride zu Verbindungen des Typs F mit R¹⁴=OH und R¹⁵=H durchgeführt. Anschließend sind durch Reaktion der Enolverbindungen der 17-Ketone F, zum Beispiel des Trialkylsilylenolethers mit Carbenen vom Typ CXY, wobei X=Y=Cl; X=Y=Br; X=F, Y=Cl; X=Cl, Y=Methyl oder X=Cl, Y=H sein kann, oder durch Simmons-Smith-Reaktion mit anschließender Öffnung der 3-Ringaddukte Verbindungen der allgemeinen Formel G mit R₁₁=F, Cl, Br, Methyl oder Wasserstoff zugänglich. Die entsprechenden Carbene CXY können zum Beispiel aus CH₃CH₂XY CHX₂Y oder CH₂XY beim Behandeln mit Basen, wie Kaliumtertiärbutanolat oder aus dem Natriumsalz der Trichloressigsäure oder der Tribromessigsäure durch Erwärmen in geeigneten Lösungsmitteln, wie zum Beispiel Dimethoxyethan/Tetrachlorethylen oder aus den Estern der Trichloressigsäure oder Tribromessigsäure zum Beispiel durch Reaktion mit Natriummethanolat erzeugt werden. Gegebenenfalls können durch Hydrierung oder Reaktion mit Trialkylzinnhydriden von G auch Verbindungen vom Typ H dargestellt werden, wobei R₁₁ hier für Methyl oder Wasserstoff steht. Die 17-Chlorverbindungen können auch nachträglich durch Reduktion mit Lithiumaluminiumhydrid oder Birch-Reaktion in die entsprechenden 17-unsubstituierten Verbindungen überführt werden.
Alternativ hierzu kann die Ringerweiterung auch über eine Tifenau- Demjenov-Umlagerung, ausgehend von den Verbindungen des Typs F erfolgen, wobei man in diesem Fall direkt zu H gelangt.

Die Verbindungen der allgemeinen Formeln G und H können nun in Verbindungen der allgemeinen Formel I überführt werden.

Hierzu werden zunächst die am 17a-C-Atom gewünschten Substituenten R² und R³ eingeführt. Diese Einführung erfolgt analog literaturbekannten Verfahren (z.B., J. Fried, J.A. Edwards, "Organic Reactions in Steroid Chemistry", Van Nostrand Reinhold Company, 1972, Vol. 1 und 2; "Terpenoids and Steroids", Specialist Periodical Report. The Chemical Society, London, Vol. 1-2) durch nucleophile Addition an das C-17a-Keton.

Die Einführung des Substituenten -C≡C-Y als R³, wobei Y die oben angegebene Bedeutung hat, erfolgt mit Hilfe einer metallierten Verbindung der allgemeinen Formel MC≡C-Y', in der Y' eine Alkin- Schutzgruppe, wie zum Beispiel Trimethylsilyl oder tert. Butyldimethylsilyl, ist.

Die metallorganische Verbindung kann auch in situ gebildet und mit dem 17-Keton zur Reaktion gebracht werden. So kann man zum Beispiel auf das 17-Keton in einem geeigneten Lösungsmittel Acetylen und ein Alkalimetall, insbesondere Kalium, Natrium oder Lithium, in Gegenwart eines Alkohols oder in Gegenwart von Ammoniak einwirken lassen. Das Alkalimetall kann auch in Form von zum Beispiel Methyl- oder Butyllithium zur Einwirkung kommen. Als Lösungsmittel sind insbesondere Dialkylether, Tetrahydrofuran, Dioxan, Benzol und Toluol geeignet.

Die Einführung von 3-Hydroxypropin, -propen in 17a-Stellung erfolgt durch Umsetzung des 17a-Ketons mit dem Dianion des Propargylalkohols 13-Hydroxypropin). zum Beispiel dem in situ generierten Dikaliumsalz des Propargylalkohols, zum 17aα-(3-Hydroxyprop-1-inyl)-17β-hydroxyderivat oder mit metallierten Derivaten des 3-Hydroxypropins, zum Beispiel mit 1-Lithium-3-(tetrahydropyran2'-yloxy)-prop-1-in-1-id, zum 17a-[3-(Tetrahydropyran-2'-yloxy)-prop-1-inyl]-17aβ-hydroxyderivat, die anschließend zu den 17a-(3-Hydroxypropyl- bzw. Hydroxypropenyl)17aβ-hydroxy-Verbindungen hydriert werden können. Das gelingt zum Beispiel durch Hydrierung bei Raumtemperatur und Normaldruck in Lösungsmitteln wie Methanol, Ethanol, Propanol, Tetrahydrofuran (THF) oder Essigester unter Zusatz von Edelmetall-Katalysatoren wie Platin oder Palladium.

Die Einführung homologer Hydroxyalkin-, Hydroxyalken- und Hydroxyalkangruppen erfolgt in entsprechender Weise mit Homologen des Propargylalkohols.

Die Verbindung mit der Z-konfigurierten Doppelbindung in der Hydroxypropenylgruppe entsteht durch Hydrieren der acetylenischen Dreifachbindung mit einem desaktivierten Edelmetallkatalysator (J. Fried, J.A. Edwards: Organic Reactions in Steroid Chemistry, Van Nostrand Reinhold Company 1972, Seite 134; und H.O. House: Modern Synthetic Reactions 1972, Seite 19).Als desaktivierte Edelmetallkatalysatoren kommen beispielsweise 10% Palladium auf Bariumsulfat in Gegenwart eines Amins oder 5 % Palladium auf Calciumcarbonat unter Zusatz von Blei(II)acetat infrage. Die Hydrierung wird nach der Aufnahme von einem Äquivalent Wasserstoff abgebrochen.

Die Verbindung mit der E-konfigurierten Doppelbindung in der Hydroxypropenylgruppe entsteht durch Reduktion der acetylenischen Dreifachbindung in an sich bekannter Weise. In der Literatur sind eine ganze Reihe von Methoden zur Umwandlung von Alkinen in trans-Olefine beschrieben, beispielsweise die Reduktion mit Natrium in flüssigem Ammoniak (J. Am. Chem. Soc. 63 (1941) 216), mit Natriumamid in flüssigem Ammoniak (J. Chem. Soc. 1955, 3558), mit Lithium in niedermolekularen Aminen (J. A. Chem. Soc. 77 (1955) 3378), mit Boranen (J. Am. Chem. Soc. 93 (1971) 3395 und 94 (1972) 6560), mit Diisobutylaluminiumhydrid und Methyl-Lithium (J. Am. Chem. Soc. 89 (1967) 5085) und insbesondere mit Lithiumaluminiumhydrid/Alkoholat (J. Am. Chem. Soc. 89 (1967) 4245). Eine weitere Möglichkeit ist die Reduktion der Dreifachbindung mit Chrom(II)-sulfat in Gegenwart von Wasser oder Dimethylformamid in schwach saurem Milieu (J. Am. Chem. Soc. 86 (1964) 4358) sowie allgemein die Reduktion durch Einwirkung von Übergangsmetallverbindungen unter Wechsel der Oxydationsstufe.

Die Einführung der Hydroxyalkene kann auch direkt erfolgen durch Addition einer entsprechenden metallierten Hydroxyalkenylverbindung, wie zum Beispiel 1-Lithium-3-(tetrahydropyran-2'-yloxy)-prop-1(E)-en (J. Org. Chem. 40 2265) oder 1Lithium-3-(tetrahydropyran-2'-yloxy)-prop-1(Z)-en (Synthesis 1981, 999) Homologe können auf diese Art ebenfalls eingeführt werden.

Die Einführung von 3-Hydroxypropan in 17a-Stellung kann ebenfalls direkt durch Umsetzung des 17a-Ketons mit metallierten Derivaten von 3-Halogen-propanolen - wobei die Hydroxygruppe im Metallierungsschritt als Alkoholat (Tetrahedron Letters 1978, 3013) oder als geschützte Funktion (J. Org. Chem. 37, 1947) vorliegt - zu der 17-(3-Hydroxypropyl)-17β-hydroxy-verbindung bzw. zu der an der terminalen Hydroxygruppe geschützten Verbindung erfolgen. Als Schutzgruppen kommmen zum Beispiel die Ethoxyethyl-, Tetrahydropyranyl- und MethoxymethylGruppen in Frage.
Werden Endprodukte der Formel I gewünscht mit R²/R³ in der Bedeutung von 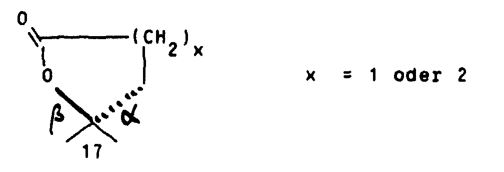 so wird die 17a-(3-Hydroxypropyl)- bzw. 17a-(4-Hydroxybutyl)-Verbindung in an sich bekannter Weise oxydiert, zum Beispiel mit Jones' Reagenz, Braunstein, Pyridiniumdichromat, Pyri- diniumchlorochromat, Chromsäure-Pyridin oder dem Fetizon-Reagenz Silbercarbonat/Celite (Compt. rend. 267 [1968] 900).
Die Darstellung von Endprodukten der Formel I mit R²/R³ in der Bedeutung von 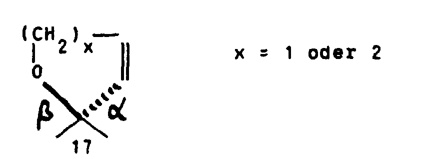 erfolgt durch Ringschlußreaktion des entsprechenden 17a-(3-Hydroxyprop-1-(Z)enyl- bzw. 17a-(4-Hydroxybut-1-(Z)-enyl-17a-β-hydroxy-Eduktes. Hydrierung des ungesättigten 5- oder 6-Ring-Spiroethers am Palladium/Aktivkohle-Kontakt führt zu den gesättigten Spiroethern.

Der Aufbau der 17a-Cyanmethylseitenkette erfolgt in an sich bekannter Weise aus dem 17a-Keton zum Beispiel über das 17a-Spiroepoxid und Spaltung des Spiroepoxids mit HCN gemäß Z. Chem. 18 (1978) 259-260.

Auch die Einführung der 17a-Hydroxyacetylseitenkette erfolgt nach an sich bekannten Methoden, beispielsweise nach den in J. Org. Chem. 47 (1982), 2993-2995, Chem.Ber. 113 (1984), 1184 bzw. US-Patent 4.600.538 beschriebenen Methoden.

Freie Hydroxygruppen können in an sich bekannter Weise alkyliert oder acyliert werden.

Der nächste Reaktionsschritt dient dem Aufbau des Substituenten R⁴ bzw. R^{4'} in p-Stellung am 11β-Phenylring.
Diese Vorgehensweise ist dann erforderlich, wenn R⁴ nicht direkt bei der Kupplung der Verbindung B mit der Arylverbindung Z zur Verbindung C eingeführt wird. Prinzipiell kann dieser Reaktionsschschritt auf jeder Stufe des Verfahrens, auch noch nach Abspaltung der Schutzgruppe(n), durchgeführt werden.
Als Ausgangspunkt für diesen Aufbau dient eine Verbindung, worin R⁴ = OH ist, die aus der entsprechenden Methoxyverbindung durch Etherspaltung, beispielsweise mit Natriumethanthiolat in einem Lösungsmittel wie Dimethylformamid, erhältlich ist.

Durch Umsetzung der Hydroxyverbindung mit einem Perfluor-(C₁-C₄)-alkylsulfonsäureanhydrid oder -halogenid in Gegenwart einer Base wie Pyridin oder 4-(Dimethylamino)-pyridin gelangt man zur entsprechenden 11β-[4-(perfluoralkylsulfonyloxy)phenyl]-Verbindung (P.J. Stang, M. Hanack und L.R. Subramanian, Synthesis 85, (1982)). Die Perfluoralkylsulfonat-Bildung kann auch schon auf einer früheren Stufe stattfinden.
Bei der sich anschließenden Kupplung der 11β-Arylverbindung mit R^{4"}-Sn(Alkyl)₃ oder R^{4"}-BL₂ wird entweder so vorgegangen, daß in einer übergangsmetallkatalysierten Reaktion (vorzugsweise Pd°) die Perfluoralkylsulfonatabgangsgruppe unter im wesentlichen fast gleichzeitiger Substitution durch den gewünschten Substituenten oder dessen Vorstufe verdrängt wird (Arylkupplungen mit Zinnverbindungen: J.E. McMurry and S. Hohanraj, Tetrahedron Letters. 24, No. 27, S. 2723-2726, 1983; X. Lu und J. Zhu, Communications, S. 726-727, 1987; Q. -Y. Chen und Z.-Y. Yang, Tetrahedron Letters 27, No. 10, S. 1171-1174, 1986; S. Cacchi. P.G. Ciattini, E. Morera und G. Ortar, Tetrahedron Letters, 27, No. 33, S. 3931-3934, 1986; A.M. Echavarren und J.K. Stille, J. Am. Chem. Soc. 1987, 109, S. 5478-5486: mit Borverbindungen: Synthesis 936 (1984), Chem.Pharm.Bull. 33,4755-4763 (1985); J.Org.Chem.49, 5237-5243 (1984); Bull.Chem.Soc. Jpn.61, 3008-3010B (1988) oder es wird aus der Perfluoralkylsulfonat-Verbindung intermediär und übergangsmetallkatalysiert eine entsprechende Tri-organylstannyl-, vorzugsweise Tri-n-alkylstannyl-Verbindung hergestellt [J.K. Stille, Angew. Chem. 98 (1986), S. 504-519]. Diese wird anschließend in einer Eintopf-Reaktion mit einem halogen-, vorzugsweise brom-oder jodsubstituierten carbocyclischen oder heterocyclischen Aromaten [Y. Yamamoto, Y. Azuma, H. Mitoh, Communications, S. 564-565, 1986; T. J. Bailey, Tetrahedron Letters, 27, No. 37, S. 4407-4410, 1986], der gegebenenfalls noch weitere Substituenten tragen kann, umgesetzt; der 11β-Phenylrest weist darin dann die gewünschte bzw. einen Vorläufer der gewünschten Substitution auf.
Zahlreiche derartige Umsetzungen mit Steroiden, in denen eine Trifluormethansulfonat-Gruppe sich in 4-Stellung eines 11β-Phenylringes befindet, sind in der EP-A-0283428 beschrieben.
Freie Hydroxygruppen können in an sich bekannter Weise alkyliert oder acyliert werden.
Dialkylamine können durch geeignete Oxidationsmittel (z.B. Wasserstoffperoxid bzw. Persäuren) in die gewünschten N-Oxide [siehe z.B. Kontakte (Darmstadt) 1986,3,S.12] überführt werden.

Verbindungen mit einem Dialkylamin-Substituenten am 11β-Phenylring können durch Reaktion mit Bromcyan in aprotischen Lösungsmitteln wie zum Beispiel Dioxan, Benzol oder Toluol bei erhöhter Temperatur (Amin-Abbau nach Braun) analog den zum Beispiel in Org. Reactions 7, 198 (1953), K.W. Bentley, Techniques of Organic Chemistry 11, 773 (1963) und Houben-Weyl, 5/4, 151 (1960) angegebenen Vorschriften in guter Ausbeute in die entsprechenden (N-Cyan-N-alkylaminoaryl)derivate überführt werden.
Diese werden je nach der letztlich gewünschten Bedeutung von 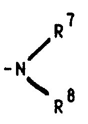 im Endprodukt in an sich bekannter Weise zu den entsprechenden Dialkylamin-Verbindungen (zum Beispiel mit Diisobutylaluminiumhydrid in Toluol zu den N-Formyl-N-alkylaminophenyl-Zwischenprodukten und anschließend mit Lithiumaluminiumhydrid) bzw. N-H-N-alkyl-Verbindungen reduziert (zum Beispiel mit Lithiumaluminiumhydrid oder mit Lithium in flüssigem Ammoniak). Die letzteren werden anschließend gewünschtenfalls in literaturbekannter Weise acyliert und gegebenenfalls anschließend in bekannter Weise mit z.B. Lithiumaluminiumhydrid zum neuen Dialkylaminderivat reduziert (s. DE 36 23 038).

Gegebenenfalls kann auch zuerst der Substituent R⁴ aufgebaut werden und anschließend die Einführung der Substituenten R² und R³ vorgenommen werden, je nachdem, ob die Verfahrensbedingungen des zweiten Reaktionsschrittes die zuerst eingeführten oder aufgebauten Substituenten beeinträchtigen.

Noch vorhandene Schutzgruppen werden nach gängigen Methoden abgespalten.

Die erhaltenen Verbindungen der allgemeinen Formel I mit X in der Bedeutung eines Sauerstoffatoms können gewünschtenfalls durch Umsetzung mit Hydroxylaminhydrochlorid in Gegenwart von tertiären Aminen bei Temperaturen zwischen -20 und +40°C in die Oxime (Formel I mit X in der Bedeutung der Hydroxyiminogruppierung 〉N∼OH, wobei die Hydroxygruppe syn- oder antiständig sein kann) überführt werden. Geeignete tertiäre Basen sind beispielsweise Trimethylamin, Triäthylamin, Pyridin, N,N-Dimethylaminopyridin, 1,5-Diazabicyclo[4.3.0]non-5en (DBN) und 1,8-Diazabicyclo[5.4.0]undec-7-en (DBU), wobei Pyridin bevorzugt ist.

Die Entfernung der 3-Oxogruppe zu einem Endprodukt der allgemeinen Formel I mit X in der Bedeutung von 2 Wasserstoffatomen kann z.B. nach der in der DE-A-2805490 angegebenen Vorschrift durch reduktive Spaltung des Thioketals erfolgen.

Die neuen Verbindungen der allgemeinen Formel I sowie deren Additionssalze mit pharmazeutisch verträglichen Säuren sind wertvolle Pharmaka. So verfügen sie über eine starke Affinität zum Gestagenrezeptor und besitzen überraschend starke antigestagene sowie antiglucocorticoide, antimineralcorticoide und antiandrogene Eigenschaften. Diese wichtigen biologischen Wirksamkeiten können für medizinische Zwecke genutzt werden.

Wirkstoffe dieser Art mit ausgeprägter antigestagener Aktivität sind zur Auslösung von Aborten geeignet, da sie das zur Aufrechterhaltung der Schwangerschaft erforderliche Progesteron vom Rezeptor verdrängen. Sie sind deshalb wertvoll und interessant im Hinblick auf ihre Verwendung zur postcoitalen Fertilitätskontrolle.

Die neuen Verbindungen können außerdem zur Behandlung der Endometriose dienen. Sie können auch gegen hormonelle Unregelmäßigkeiten, zur Menstruationsauslösung und zur Geburtseinleitung eingesetzt werden. Außerdem können sie für die Behandlung von hormonabhängigen Carcinomen eingesetzt werden.

Die erfindungsgemäßen Verbindungen der allgemeinen Formel I sowie deren Additionssalze mit pharmazeutisch verträglichen Säuren weisen auch eine antiglucocorticoide Aktivität auf und können somit auch als Arzneimittel zur Therapie corticoid-induzierter Störungen (Glaukom) sowie zur Bekämpfung von Nebenwirkungen, die bei langfristiger Behandlung mit Glucocorticoiden auftreten (Cushing-Syndrom) eingesetzt werden. Sie ermöglichen daher auch die auf eine Supersekretion der Glucocorticoide zurückzuführenden Störungen, vor allem die Adipositas, Arteriosklerose, Hypertension, Osteoporose, Diabetes sowie die Insomnie zu bekämpfen.

Die erfindungsgemäßen Verbindungen der allgemeinen Formel I sowie deren Additionssalze mit pharmazeutisch verträglichen Säuren mit antiandrogener Aktivität können bei der Behandlung der Hypertrophie und des Prostatakarzinoms verwendet werden. Sie ermöglichen weiterhin eine spezifische Therapie von Androgenisierungserscheinungen bei Frauen: die pathologische Behaarung bei Hirsutismus, die androgenetische Alopezie sowie die gesteigerte Talgdrüsenfunktion bei Akne und Seborrhoe sind günstig beeinflußbar.

Die Erfindung betrifft somit auch Arzneimittel auf Basis der Verbindungen der allgemeinen Formel I sowie deren Additionssalze mit pharmazeutisch verträglichen Säuren, gegebenenfalls zusammen mit den üblichen Hilfs- und Trägerstoffen.

Die erfindungsgemäßen Verbindungen und deren Salze können nach an sich bekannten Methoden der Galenik zu pharmazeutischen Präparaten für die enterale, perkutane, parenterale oder lokale Applikation verarbeitet werden. Sie können in Form von Tabletten, Dragees, Gelkapseln, Granulaten, Suppositorien, Implantaten, injizierbaren sterilen wäßrigen oder öligen Lösungen, Suspensionen oder Emulsionen, Salben, Cremes und Gelen verabreicht werden.

Der oder die Wirkstoffe können dabei mit den in der Galenik üblichen Hilfsstoffen wie z.B. Gummiarabikum, Talk, Stärke, Mannit, Methylcellulose, Laktose, Tensiden wie Tweens^{(R)} oder Myrj^{(R)}, Magnesiumstearat, wäßrigen oder nichtwäßrigen Trägern, Paraffinderivaten, Netz-, Dispergier-, Emulgier-, Konservierungsmitteln und Aromastoffen zur Geschmackskorrektur (z.B. ätherischen Ölen) gemischt werden.

Die Erfindung betrifft somit auch pharmazeutische Zusammensetzungen, die als Wirkstoff zumindest eine erfindungsgemäße Verbindung oder eines ihrer Additionssalze mit pharmazeutisch verträglichen Säuren enthalten. Als Additionssalze der erfindungsgemäßen Produkte mit Säuren sind insbesondere die Hydrochloride und die Methansulfonate zu nennen. Eine Dosiseinheit enthält etwa 1-100 mg Wirkstoff(e).
Die Dosierung der erfindungsgemäßen Verbindungen liegt beim Menschen bei etwa 1-1000 mg pro Tag.

Pharmakologische Untersuchungen:

Die starke Affinität zum Gestagenrezeptor ergibt sich aus dem bekannten, u.a. in der EP-A-0 190 759 beschriebenen Gestagenrezeptor-Bindungstest. Folgende Verbindungen wurden untersucht:
17-Chlor-11β-(4-methoxyphenyl)-17aβ-hydroxy-17aα-(1-propinyl)-17a-homoestra-4,16-dien-3-on (A)
17-Chlor-17aβ-hydroxy-17aα-(1-propinyl)-11β-[4-(3-pyridinyl)phenyl]-17a-homoestra-4,16-dien-3-on (B)
17-Chlor-11β-[4-(3-furanyl)phenyl]-17aβ-hydroxy-17aα-methyl-17a-homoestra-4,16-dien-3-on (C)

Die untersuchten Verbindungen weisen folgende Kompetitionsfaktoren auf (Bezugsubstanz: ³H-Progesteron; Gewebe aus Kaninchenuterus),

| | | | |
|---|---|---|---|
| Testverbindung | A | B | C |
| Kompetitionsfaktor K | 1,5 | 3,5 | 4,5 |

Zur Kennzeichnung der antigestagenen Wirkung wurde die abortive Wirkung an graviden Ratten nach dem in der EP-A-0 283 428 beschriebenen Test bestimmt.

Untersucht wurden die Verbindungen A, B und C (siehe Tabelle 1). Applikation der Testverbindungen an d5-d7 p.c. p.o.; Autopsie an d9 p.c.

Die nachfolgenden Beispiele dienen der näheren Erläuterung der Erfindung:

### Beispiel 1

### Herstellung von 17-Chlor-11β-(4-methoxyphenyl)-17aβ-hydroxy-17aα-(1-propinyl)-17a-homoestra-4,16-dien-3-on (1K)

A) 3,3;17,17-Bis[1,2-ethandiylbis(oxy)]-11-[[(trifluormethyl)sulfonyl]oxy]estra-5,9(11)-dien (1A)
   26,1 g 3,3;17,17-Bis[1,2-ethandiylbis(oxy)]estr-5-en-11-on werden in 350 ml absolutem Methylenchlorid gelöst und unter Schutzgas mit 18 ml 2,6-Di-tertiär-butylpyridin versetzt. Nach Kühlen dieser Lösung auf 0°C werden 12,9 ml Trifluormethansulfonsäureanhydrid langsam zugetropft. Danach wird das Reaktionsgemisch 20 Stunden bei Raumtemperatur nachgerührt. Zur Aufarbeitung wird es auf gesättigte Natriumhydrogencarbonatlösung gegossen, die organische Phase abgetrennt und die wäßrige mit Methylenchlorid nachextrahiert. Die vereinigten organischen Phasen werden mit gesättigter Natriumchloridlösung gewaschen, über Natriumsulfat getrocknet und am Vakuum eingeengt. Nach Chromatographie des Rohprodukts an Kieselgel mit einem Gemisch aus Ethylacetat/Hexan werden neben 16,4 ml 2,6-Di-tertiär-butylpyridin und 5,1 g 3,3;17,17-Bis[1,2-ethandiylbis(oxy)]estr-5-en-11-on 27 g der Titelverbindung 1A als weißer Schaum erhalten.
   [α]²⁰_{D} = + 104 ° (CHCl₃;c=0.505)
   ¹H-NMR(CDCl₃) δ: 5,58 dbr (J=5 Hz,1H,H-6); 3,7-4,0 m (8H,Ketal); 2,88 dbr (J=11 Hz,1H, H-10); 2,74 dtr (J=16, 2.5 Hz,1H,H-12); 2,18-2,33 m (2H,H-4); 0,84 s (3H,H-18).
B) 3,3;17,17-Bis[1,2-ethandiylbis(oxy)]-11-(4-methoxyphenyl)estra-5,9(11)-dien (1B)
   27 g 1A werden in einem Gemisch aus 450 ml absolutem Toluol und 210 ml absolutem Ethanol gelöst und nacheinander mit 3,1 g Palladiumtetrakistriphenylphosphin, 4,5 g Lithiumchlorid, 70 ml 2 molarer Natriumcarbonatlösung und 9 g (4-Methoxyphenyl)boronsäure versetzt. Das Reaktionsgemisch wird dann 2 Stunden bei 95 °C gerührt, auf Raumtemperatur abgekühlt und mit gesättigter Natriumchloridlösung versetzt. Die organische Phase wird abgetrennt, nacheinander mit 5 %-iger Natronlauge und Wasser gewaschen, über Natriumsulfat getrocknet und im Vakuum eingeengt. Der Rückstand wird an Kieselgel mit einem Gemisch aus Ethylacetat/Hexan chromatographiert. Man erhält 24 g 1B als weißen Schaum.
C) 3,3;17,17-Bis[1,2-ethandiylbis(oxy)]-11β-(4-methoxyphenyl)estr-3-en (1C)
   1000 ml Ammoniak werden bei -70 °C kondensiert und mit 1,80 g Lithium versetzt. Nach Auftreten der charakteristischen Blaufärbung werden 24 g 1B gelöst in 500 ml absolutem Tetrahydrofuran zugetropft. Nach 20-minütigem Nachrühren zersetzt man das überschüssige Lithium durch Zugabe von Wasser, dampft den Ammoniak ab, gießt das Reaktionsgemisch auf gesättigte Ammoniumchloridlösung und extrahiert die wäßrige Phase mit Ethylacetat. Die vereinigten organischen Phasen werden mit gesättigter Natriumchloridlösung gewaschen, über Natriumsulfat getrocknet und am Vakuum eingeengt. Nach Chromatographie des Rohprodukts an Kieselgel mit einem Gemisch aus Etylacetat/Hexan werden 19,6 g der Titelverbindung 1C und 1,8 g 3,3;17,17-Bis[1,2-ethandiylbis(oxy)]-11-(4-hydroxyphenyl)estra-5,9(11)-dien als weiße Schäume isoliert.
D) 3,3-[1,2-Ethandiylbis(oxy)]-11β-(4-methoxyphenyl)estr-5-en-17-on (1D)
   59 g Kieselgel werden in 130 ml Methylenchlorid suspendiert mit 5,9 ml gesättigter Oxalsäurelösung versetzt und 15 Minuten nachgerührt. Zu dieser Suspension werden 19,6 g 1C gegeben und das Reaktionsgemisch bei Raumtemperatur 4 Stunden nachgerührt. Anschließend wird es über eine Fritte abgesaugt, der Frittenrückstand mit Methanol/methylenchlorid nachgewaschen und das so erhaltene Filtrat mit gesättigter Natriumhydrogencarbonatlösung ausgeschüttelt. Die organische Phase wird über Natriumsulfat getrocknet und am Vakuum eingeengt. Der Rückstand wird an Kieselgel mit einem Gemisch aus Ethylacetat/Hexan chromatographiert. Man erhält 13,77 g 1D als weißen Schaum.
E) 3,3-[1,2-Ethandiylbis(oxy)]-11β-(4-methoxyphenyl)-5,6α-epoxy-5α-estran-17-on (1E)
   4,75 g 1D werden in 35 ml Methylenchlorid gelöst. Man versetzt mit 3,8 ml gesättigter Natriumhydrogencarbonatlösung, kühlt auf 0°C und gibt 1,23 g *m*-Nitrotrifluoracetophenon hinzu. Anschließend werden 4,66 ml einer 30%igen Wasserstoffperoxidlösung hinzugetropft. Es wird 4 Tage bei Raumtemperatur nachgerührt. Dann wird die Reaktionslösung vorsichtig bei leichter Kühlung mit gesättigter Natriumthiosulfatlösung versetzt. Man extrahiert mit Methylenchlorid, wäscht die organische Phase mit 5%iger Natriumhydroxidlösung sowie mit gesättigter Natriumchloridlösung und trocknet über Natriumsulfat. Es wird im Vakuum eingeengt. Das Rohprodukt wird durch Säulenchromatographie gereinigt. Man erhält 3,74 g 1E.
   ¹H-NMR (CDCl₃): δ= 7,24 d (J=10 Hz, 2H, Ar); 6,79 d (J=10 Hz, 2H, Ar); 3,72-4,02 m (4H, Ketal); 3,80 s (3H, OMe); 3,25 ddbr (J=7, 5 Hz, 1H, H-11); 2,97 d (J=5 Hz, 1H, H-6); 0,60 s (3H, H-18)
F) 3,3-[1,2-Ethandiylbis(oxy)]-11β-(4-methoxyphenyl)-5α-estra-5,17β-diol (1F)
   3,74 g 1E werden in 250 ml absolutem Ethanol gelöst. Man gibt vorsichtig 6,45 g Natriumborhydrid hinzu und kocht 1 Stunde unter Rückfluß. Anschließend wird die Reaktionslösung auf Wasser gegossen. Man extrahiert mit Methylenchlorid, wäscht die organische Phase mit gesättigter Natriumchloridlösung, trocknet über Natriumsulfat und engt im Vakuum ein. Man erhält 3,99 g 1F, welches als Rohprodukt in die nächste Stufe eingesetzt wird.
   ¹H-NMR (CDCl₃): δ= 7,29 d (J=10 Hz, 2H, Ar); 6,78 d (J=10 Hz, 2H, Ar); 3,85-3,97 m (4H, Ketal); 3,70 s (3H, OMe); 3,58 ddbr (J=14, 7 Hz, 1H, H-17); 3,12 m (1H, H-11); 0,47 s (3H, H-18)
G) 3,3-[1,2-Ethandiylbis(oxy)]-5-hydroxy-11β-(4-methoxyphenyl)-5α-estran-17-on (1G)
   Zu 7,9 ml Pyridin in 30 ml Methylenchlorid gibt man bei 0°C 2,36 g Chromtrioxid und läßt 30 Minuten nachrühren. Dann wird eine Lösung von 1,74 g 1F in 6 ml Methylenchlorid hinzugetropft und, 1 Stunde bei 0°C nachgerührt. Die Reaktionslösung wird anschließend mit 5%iger Natriumhydroxidlösung, sowie gesättigter Natriumchloridlösung gewaschen. Es wird über Natriumsulfat getrocknet und im Vakuum eingeengt. Das Rohprodukt wird durch Kristallisation aus Diisopropylether gereinigt. Man erhält 1,21 g der Titelverbindung 1G.
   ¹H-NMR (CDCl₃): δ= 7,29 d (10 Hz, 2H, Ar); 6,78 d (J=10 Hz, 2H, Ar); 3,85-4,00 m (4H, Ketal); 3,78 s (3H, OMe); 3,18 m (1H, H-11); 0,58 s (3H, H-18)
H) 3,3-[1,2-Ethandiylbis(oxy)]-11β-(4-methoxyphenyl)-17-[(trimethylsilyl)oxy]-5α-estr-16-en-5-ol (1H)
   Aus 1,10 ml Diisopropylamin und 4,8 ml einer 1,6 molaren Butyllithiumlösung (in Hexan) wird in absolutem Tetrahydrofuran bei -30°C Lithiumdiisopropylamin erzeugt. Dann tropft man bei -40°C 1,21 g 1G in 10 ml absolutem Tetrahydrofuran hinzu und rührt 1 Stunde nach. Anschließend werden 1,04 ml Trimethylsilylchlorid hinzugetropft. Man rührt 30 Minuten bei Raumtemperatur nach, gießt dann die Reaktionslösung auf gesättigte Natriumhydrogencarbonatlösung und extrahiert mit Ethylacetat. Die organische Phase wird mit gesättigter Ammoniumchloridlösung sowie gesättigter Natriumchloridlösung gewaschen, über Natriumsulfat getrocknet und eingeengt. Das so erhaltene 1H wird direkt weiter eingesetzt.
I) 17-Chlor-3,3-[1,2-ethandiylbis(oxy)]-5-hydroxy-11β-(4-methoxyphenyl)-17a-homo-5α-estr-16-en-17a-on (1I)
   Hierzu löst 1H man in einer Mischung aus 2,5 ml Ethylenglycoldimethylether und 7,55 ml Tetrachlorethylen, versetzt mit 980 mg Natriumtrichloracetat und kocht 20 Stunden unter Rückfluß (nach ca. 8 Stunden werden weitere 980 mg Natriumtrichloracetat hinzugefügt). Anschließend wird die Reaktionslösung auf Wasser gegossen. Man extrahiert mit Methylenchlorid. Die organische Phase wird mit gesättigter Ammoniumchloridlösung sowie gesättigter Natriumchloridlösung gewaschen, über Natriumsulfat getrocknet und im Vakuum eingeengt. Das Rohprodukt wird durch Säulenchromatographie an Kieselgel mit einem Gemisch aus Hexan/Ethylacetat gereinigt. Man erhält 800 mg 1I.
   ¹H-NMR (CDCl₃): δ= 7,31 d (J=10 Hz, 2H, Ar); 6,98 dd (J=6, 1.5 Hz, 1H, H-16); 6,79 d (J=10 Hz, 2H, Ar); 3,85-3,98 m (4H, Ketal); 3,22 ddbr (J=7, 5 Hz, 1H, H-11); 2,64 ddd (J=19, 5.5, 4.5 Hz, 1H, H-15β); 2,38 dd (J=15, 1.5 Hz, 1H, H-12β); 2,08 dd (J=19, 2.5 Hz, 1H, H-15α); 1,95 dd (J=15, 5.5, 1H, H-12α); 0,73 s (3H, H-18)
K) 17-Chlor-3,3-[1,2-ethandiylbis(oxy)]-11β-(4-methoxyphenyl)-17aα-(1-propinyl)-17a-homo-5α-estr-16-en-5,17aβ-diol (1K)
   In 130 ml absolutes Tetrahydrofuran wird bei 0°C über 40 Minuten Propin geleitet. Der Reaktionskolben wird mit Argon gespült und anschließend werden bei -8°C 10,3 ml einer 1,6 molaren Butyllithiumlösung in Hexan hizugetropft. Man rührt 30 Minuten nach und tropft dann 800 mg 1I, gelöst in 8,2 ml THF, hinzu. Es wird 2 Stunden bei 0°C nachgerührt. Danach werden 30 ml gesättigte Ammoniumchloridlösung dazugetropft. Man extrahiert mit Ethylacetat, wäscht die organische Phase mit gesättigter Natriumchloridlösung, trocknet über Natriumsulfat und engt im Vakuum ein. Man erhält 781 mg 1K. Das Rohprodukt wird ohne Reinigung in die nächste Stufe eingesetzt.
   ¹H-NMR (CDCl₃): δ= 7,31 d (J=10 Hz, 2H, Ar); 6,78 d (J=10 Hz, 2H, Ar); 5,79 dd (J=5, 2.5 Hz, 1H, H-16); 3,86-4,00 m (4H, Ketal); 3,80 s (3H, OMe); 3,22 ddbr (J=7, 5 Hz, 1H, H-11); 2,53 dd (J=14, 6.5 Hz, 1H, H-15β); 2,13 dd (J=14, 2.5 Hz, 1H, H-15α); 1,93 s (3H, Me); 0,56 s (3H, H-18)
L) 17-Chlor-11β-(4-methoxyphenyl)-17aβ-hydroxy-17aα-(1-propinyl)-17a-homoestra-4,16-dien-3-on (1L)
   779 mg 1K werden in 26 ml Aceton gelöst. Man versetzt mit 1,95 ml 2 molarer wäßriger Salzsäure und rührt 2 Stunden bei Raumtemperatur. Anschließend wird die Reaktionslösung auf gesättigte Natriumhydrogencarbonatlösung gegossen und mit Methylenchlorid extrahiert. Die organische Phase wird mit gesättigter Natriumchloridlösung gewaschen, über Natriumsulfat getrocknet und Vakuum eingeengt. Das Rohprodukt wird durch Säulenchromatographie an Kieselgel mit einem Gemisch aus Hexan und Ethylacetat gereinigt. Man erhält 435 mg der Titelverbindung 1L als weißen Schaum.
   ¹H-NMR (CDCl₃): δ= 7,35 d (J=10 Hz, 2H, Ar); 6,83 d (J=10 Hz, 2H, Ar); 5,86 sbr (1H, H-4); 5,80 dd (J=5, 1 Hz, 1H, H-16); 3,80 s (3H, OMe); 3,39 ddbr (J=7, 5 Hz, 1H, H-11); 2,89 m (1H, H-10); 1,92 s (3H, Me); 0,68 s (3H, H-18)
   [α]²⁰_{D}= -120° (CHCl₃; c=0,510)

### Beispiel 2

### Herstellung von 17-Chlor-17aβ-hydroxy-17aα-(1-propinyl)-11β-[4-(3-pyridinyl)phenyl]-17a-homoestra-4,16-dien-3-on (2G)

A) 3,3-[1,2-Ethandiylbis(oxy)]-11β-(4-hydroxyphenyl)-5α-estra-5,17β-diol (2A)
   2,41 g 1F werden in 25 ml absolutem Dimethylformamid gelöst. Man versetzt im Argongegenstrom mit 1,53 g Natriummethanthiolat und kocht 2 Stunden unter Rückfluß. Anschliessend wird die Reaktionslösung auf ca. 50 ml Eiswasser gegossen. Man extrahiert mit Ethylacetat, wäscht die organische Phase mit gesättigter Natriumchloridlösung, trocknet über Natriumsulfat und engt im Vakuum ein. Das Rohprodukt wird durch Säulenchromatographie an Kieselgel mit einem Gemisch aus Hexan/Ethylacetat gereinigt. Man erhält 1,37 g 2A als weißen Schaum.
   ¹H-NMR (CDCl₃): δ= 7,23 d (J=10 Hz, 2H, Ar); 6,71 d (J=10 Hz, 2H, Ar); 3,85-3,97 m (4H, Ketal); 3,58 dd (J=12, 5 Hz, 1H, H-17); 3,12 m (1H, H-11); 0,46 s (3H, H-18)
B) 3,3-[1,2-Ethandiylbis(oxy)]-11β-[4-[[(trifluormethyl)sulfonyl]oxy]phenyl]-5α-estra-5,17β-diol (2B)
   1,37 g 2A werden in 50 ml absolutem Methylenchlorid gelöst. Man versetzt mit, 1,94 g Dimethylaminopyridin, kühlt auf -78°C und tropft 0,7 ml Trifluormethansulfonsäureanhydrid hinzu. Anschließend läßt man 2 Stunden bei -78°C rühren und gießt dann auf gesättigte Natriumhydrogencarbonatlösung. Man rührt 30 Minuten nach und extrahiert mit Methylenchlorid. Die organische Phase wird mit gesättigter Natriumchloridlösung gewaschen, über Natriumsulfat getrocknet und im Vakuum eingeengt. Das Rohprodukt wird durch Säulenchromatographie an Kieselgel mit einem Gemisch aus Hexan und Ethylacetats gereinigt. Man erhält 1,14 g 2B als weißen Schaum.
   ¹H-NMR (CDCl₃): δ= 7,48 d (J=10 Hz, 2H, Ar); 7,16 d (J=10 Hz, 2H, Ar); 3,84-3,97 m (4H, Ketal); 3,58 dd (J=12.5, 5 Hz, 1H, H-17); 3,21 ddbr (J=7, 5 Hz, 1H, H-11); 0,41 s (3H, H-18)
C) 3,3-[1,2-Ethandiylbis(oxy)]-5-hydroxy-11β-[4-[[(trifluormethyl)sulfonyl]oxy]phenyl]-5α-estran-17-on (2C)
   Zu 4,07 ml Pyridin in 15 ml Methylenchlorid gibt man 1,22 g Chromtrioxid und rührt 15 Minuten bei 0°C nach. Anschließend werden 1,14 g 2B in 5 ml Methylenchlorid hinzugetropft. Man läßt eine weitere Stunde bei 0°C rühren. Die Reaktionslösung wird dann mit 5%iger wäßriger Natriumhydroxidlösung, sowie mit gesättigter Natriumchloridlösung gewaschen, über Natriumsulfat getrocknet und im Vakuum eingeengt. Das Rohprodukt wird durch Säulenchromatographie an Kieselgel mit einem Gemisch aus Hexan und Ethylacetat gereinigt. Man erhält 1,03 g 2C.
   ¹H-NMR (CDCl₃): δ= 7,48 d (J=10 Hz, 2H, Ar); 7,18 d (J=10, 2H, Ar); 3,85-3,98 m (4H, Ketal); 3,28 ddbr (J=7, 5 Hz, 1H, H-11); 0,54 s (3H, H-18)
D) 17-Chlor-3,3-[1,2-ethandiylbis(oxy)]-5-hydroxy-11β-[4-[[(trifluormethyl)sulfonyl]oxy]phenyl]-17a-homo-5α-estr-16-en-17a-on (2D)
   Aus 0,78 ml Diisopropylamin und 3,46 ml einer 1,6 molaren Lösung von Butyllithium in Hexan sowie 1,03 g 2C wird zunächst analog zu Beispiel 1H) 3,3-[1,2-Ethandiylbis(oxy)]-11β-[4[[(trifluormethyl)sulfonyl]oxy]phenyl]-17-[(trimethylsilyl)oxy]-5α-estr-16-en-5-ol hergestellt. Dann werden durch Rückflußkochen mit 660 mg Natriumtrichloracetat in einer Mischung aus 1,9 ml Ethylenglycoldimethylether und 5,2 ml Tetrachlorethylen 387 mg 2D dargestellt.
   ¹H-NMR (CDCl₃): δ= 7,51 d (J=10 Hz, 2H, Ar); 7,18 d (J=10 Hz, 2H, Ar); 7,01 dd (J=6, 2 Hz, 1H, H-16); 3,85-4,00 m (4H, Ketal); 3,31 ddbr (J=7, 5 Hz, 1H, H-11); 2,66 ddd (J=19, 6, 4.5 Hz, 1H, H-15β); 2,39 dd (J=15, 1.5 Hz, 1H, H-12β); 2,12 dd (J=19, 2.5 Hz, 1H, H-15α); 2,01 dd (J=15, 6 Hz, 1H, H-12α)
E) 17-Chlor-3,3-[1,2-ethandiylbis(oxy)]-17aα-(1-proninyl)-11β-[4-[[(trifluormethyl)sulfonyl]oxy]phenyl]-17a-homo-5α-estr-16-en-5,17aβ-diol (2E)
   Wie in Beispiel 1K) beschrieben werden aus 4 ml einer 1,6 molaren Lösung von Butyllithium in Hexan, 387 mg 2D und Propin in 25 ml absolutem Tetrahydrofuran 408 mg 2E dargestellt. Das Rohprodukt wird ohne Reinigung weiter eingesetzt.
   ¹H-NMR (CDC₃): δ= 7,49 d (J=10 Hz, 2H, Ar); 7,16 d (J=10 Hz, 2H, Ar); 5,38 dd (J=5, 2.5 Hz, 1H, H-16); 3,85-4,00 m (4H, Ketal); 3,40 ddbr (J=7, 5 Hz, 1H, H-11); 2,59 dd (J=14, 6 Hz, 1H, H-15β); 2,16 dd (J=14, 2.5 Hz, 1H, H-15α); 1,92 s (3H, Me), 0,54 s (3H, H-18)

Die Verbindung 2E läßt sich auch auf folgendem Wege herstellen:
AA) 3,3-[1,2-Ethandiylbis(oxy)]-11β-(4-hydroxyphenyl)estr-5-en-17-on (2AA)
   Analog zu Beispiel 2A) werden 20 g 1D und 13,4 g Natriummethanthiolat in 350 ml Dimethylformamid umgesetzt. Nach beendeter Reaktion wird auf 600 ml Eiswasser gegossen und über Nacht nachgerührt. Es wird abgesaugt und das Filtrat mehrmals mit Wasser gewaschen. Man erhält 19 g 2AA, die ohne Reinigung in die Folgestufe eingesetzt werden.
AB) 11β-[4-[[Dimethyl(1,1-dimethylethyl)silyl]oxy]phenyl]-3,3-[1,2-ethandiylbis(oxy)]estr-5-en-17-on (2AB)
   8,9 g 2AA werden in 90 ml Dimethylformamid gelöst. Man addiert 4,63 g 1*H*-Imidazol und 4,93 g Dimethyl(1,1-dimethylethyl)silylchlorid und rührt 4 Stunden bei Raumtemperatur nach. Anschließend wird die Reaktionslösung auf Eiswasser gegossen und mit Ethylacetat extrahiert. Man wäscht die organische Phase mit gesättigter wäßriger Natriumchloridlösung, filtriert und engt am Vakuum ein. Das erhaltene Rohprodukt wird durch Säulenchromatographie an Kieselgel mit einem Gcmisch aus Hexan/Ethylacetat gereinigt. Man erhält 10,27 g 2AB als weißen Schaum.
   ¹H-NMR (CDCl₃): δ= 7,09 d (J=8 Hz,2H,Ar); 6,63 d (J=8 Hz,2H,Ar); 5,46-5,48 m (1H,H-6); 3,80-3,90 m (4H,Ketal); 3,30 ddbr (J=5, 7 Hz,1H,H-11); 0,88 s (9H,t-Bu); 0,50 s (3H,C-18); 0,10 s (6H,SiMe₂)
AC)11β-[4-[[Dimethyl(1,1-dimethylethyl)silyl]oxy]phenyl]-3,3-[1,2-ethandiylbis(oxy)]-5,6α-epoxy-5α-estran-17-on (2AC)
   Wie unter Beispiel 1E) beschrieben werden 2,63 g 2AB, 549 mg 3-Nitrotrifluoracetophenon, 209 ml 30%iges Wasserstoffperoxid und 1,7 ml gesättigte wäßrige Natriumhydrogencarbonatlösung in 20 ml Dichlormethan umgesetzt. Man erhält nach Säulenchromatographie an Kieselgel mit einem Gemisch aus Hexan/Ethylacetat 1,9 g 2AC als weißen Schaum.
   ¹H-NMR (CDCl₃): δ= 7,19 d (J=8 Hz,2H,Ar); 6,72 d (J=8 Hz,2H,Ar); 3,80-4,00 m (4H,Ketal); 3,22 ddbr (J=5, 7 Hz,1H,H-11); 2,96 d (J=5 Hz,1H,H-6); 0,96 s (9H,t-Bu); 0,60 s (3H, C-18); 0,20 s (6H,SiMe₂)
AD) 11β-[4-[[Dimethyl(1,1-dimethylethyl)silyl]oxy]phenyl]-3,3-[1,2-ethandiylbis(oxy)]-5α-estra-5,17β-diol (2AD)
   Zu 550 mg Lithiumaluminiumhydrid in 20 ml absolutem Tetrahydrofuran werden bei 0°C 3,85 g 2AC, gelöst in 30 ml absolutem Tetrahydrofuran, addiert. Man läßt 30 Minuten bei 0°C nachrühren und addiert dann vorsichtig 5 ml gesättigte wäßrige Ammoniumchloridlösung. Der ausgefallene Niederschlag wird über Celite abfiltriert. Das Filtrat wird mit Ethylacetat verdünnt und mit gesättigter wäßriger Natriumchloridlösung gewaschen. Man trocknet über Natriumsulfat, filtriert und engt am Vakuum ein. Nach Säulenchromatographie an Kieselgel mit einem Gemisch aus Hexan/Ethylacetat werden 3,26 g 2AD als weißer Schaum erhalten.
   ¹H-NMR (CDCl₃): δ= 7,20 d (J=8 Hz,2H,Ar); 6,70 d (J=8 Hz,2H,Ar); 3,85-4,00 m (4H,Ketal); 3,55 m (1H,H-17); 3,10 ddbr (J=5, 7 Hz,1H,H-11); 0,98 s (9H,t-Bu); 0,48 s (3H,C-18); 0,18 s (6H,SiMe₂)
AE) 11β-[4-[[Dimethyl(1,1-dimethylethyl)silyl]oxy]phenyl]-3,3-[1,2-ethandiylbis(oxy)]-5-hydroxy-5α-estran-17-on (2AE)
   3,37 g 2AD, 3,73 g Chromtrioxid und 1,24 ml Pyridin werden analog zu Beispiel 1G) umgesetzt. Man erhält nach Säulenchromatographie an Kieselgel mit einem Gemisch aus Hexan/ Ethylacetat 3,23 g 2AE als weißen Schaum.
   ¹H-NMR (CDCl₃): δ= 7,20 d (J=8 Hz,2H,Ar); 6,70 d (J=8 Hz,2H,Ar); 3,85-3,95 m (4H,Ketal); 3,15 ddbr (J=5, 7 Hz,1H,H-11); 0,95 s (9H,t-Bu); 0,57 s (3H,C-18); 0,15 s (6H,SiMe₂)
AF) 11β-[4-[[Dimethyl(1,1-dimethylethyl)silyl)oxy]phenyl]-3,3-[1,2-ethandiylbis(oxy)]-17-[(trimethylsilyl)oxy]-5α-estr-16-on-5-ol (2AF)
   Aus 1,08 g 2AE, 0,7 ml Diisopropylamin, 3,13 ml einer 1,6 molaren Lösung von Butyllithium in Hexan sowie 0,7 ml Trimethylsilylchlorid werden in 60 ml absolutem Tetrahydrofuran analog zu Beispiel 1H) 1,16 g 2AF dargestellt, welche ohne Reinigung in die Folgestufe eingesetzt werden.
AG) 17-Chlor-11β-[4-[[dimethyl(1,1-dimethylethyl)silyl]oxy]phenyl]-3,3-[1,2-ethandiylbis-(oxy)]-5-hydroxy-17a-homo-5α-estr-16-en-17a-on (2AG)
   1,16 g 2AF werden in 10 ml Trichlormethan gelöst. Man versetzt mit 10 mg Benzyltriethylammoniumchlorid sowie 2,1 g Natriumtrichloracetat und kocht 3 Stunden unter Rückfluß. Anschließend wird das Reaktionsgemisch auf Wasser gegossen. Man extrahiert mit Dichlormethan, wäscht die organische Phase mit gesättigter wäßriger Ammoniumchloridlösung sowie gesättigter wäßriger Natriumchloridlösung, trocknet über Natriumsulfat, filtriert und engt am Vakuum ein. Man erhält nach Säulenchromatographie an Kieselgel mit einem Gemisch aus Hexan/Ethylacetat 694 mg 2AG als weißen Schaum.
   ¹H-NMR (CDCl₃): δ= 7,25 d (J=8 Hz,2H,Ar); 6,98 dd (J=6, 1.5 Hz,1H,H-16); 6,73 d (J=8 Hz,2H,Ar); 3,90-4,00 m (4H,Ketal); 3,20 ddbr (J=5, 7 Hz,1H,H-11); 0,95 s (9H,t-Bu); 0,73 s (3H,C-18); 0,18 s (6H, SiMe₂)
AH) 17-Chlor-11β-[4-[[dimethyl(1,1-dimethylethyl)silyl)oxy]phenyl]-3,3-[1,2-ethandiylbis-(oxy)]-17aα-(1-propinyl)-17a-homo-5α-estr-16-en-5,17aβ-diol (2AH)
   Analog zu Beispiel 1K) werden 694 mg 2AG, 3,7 ml einer 1,6 molaren Lösung von Butyllithium in Hexan und Propingas in 30 ml absolutem Tetrahydrofuran umgesetzt. Man erhält 652 mg Rohprodukt 2AH, welches ohne Reinigung in die Folgestufe eingesetzt wird.
AI) 17-Chlor-3,3-[1,2-ethandiylbis(oxy)]-11β-(4-hydroxyphenyl)-17aα-(1-propinyl)-17a-homo-5α-estr-16-en-5,17aβ-diol (2AI)
   652 mg 2AH werden in 10 ml absolutem Tetrahydrofuran gelöst. Man addiert 820 Tetrabutylammoniumfluorid-trihydrat und rührt 1,5 Stunden bei Raumtemperatur nach. Anschliessend wird die Reaktionslösung auf gesättigte wäßrige Natriumhydrogencarbonatlösung gegossen und mit Ethylacetat extrahiert. Die organische Phase wird mit gesättigter wäßriger Natriumchloridlösung gewaschen, über Natriumsulfat getrocknet, filtriert und am Vakuum eingeengt. Man erhält 530 mg 2AI, welches ohne Reinigung weiter eingesetzt wird.
AK) 17-Chlor-3,3-[1,2-ethandiylbis(oxy)]-17aα-(1-propinyl)-11β-[4[[(trifluormethyl)sulfonyl]oxy]phenyl]-17a-homo-5α-estr-16-en-5,17aβ-diol (2E)
   Analog zu Beispiel 2B) werden 530 mg 2AI, 0,23 ml Trifluormethansulfonsäureanhydrid und 0,74 g 4-Dimethylaminopyridin in 15 ml absolutem Dichlormethan umgesetzt. Man erhält nach Säulenchromatographie an Kieselgel mit einem Gemisch aus Hexan/Ethylacetat 504 mg 2E als weißen Schaum.
F) 17-Chlor-3,3-[1,2-ethandiylbis(oxy)]-17aα-(1-propiny)-11β-[4-(3-pyridinyl)phenyl]-17a-homo-5α-estr-16-en-5,17aβ-diol (2F)
   1,28 g 2E werden in 18 ml Toluol und 8 ml Ethanol gelöst. Man addiert unter Argon 408 mg Diethyl(3-pyridinyl)boran, 235 mg Tetrakis(triphenylphosphin)palladium, 170 mg Lithiumchlorid sowie 2,6 ml einer 2 molaren wäßrigen Natriumcarbonatlösung und kocht 1,5 Stunden unter Rückfluß. Anschließend wird die Reaktionslösung auf Wasser gegossen und mit Ethylacetat extrahiert. Man wäscht die organische Phase mit gesättigter wäßriger Natriumchloridlösung, trocknet über Natriumsulfat, filtriert und engt am Vakuum ein. Das Rohprodukt wird an Kieselgel mit einem Gemisch aus Ethylacetat/Hexan chromatographiert. Man erhält 925 mg 2F als weißen Schaum.
   ¹H-NMR (CDCl₃): δ= 8,87 sbr (1H,Py); 8,56 dbr (J=4.5 Hz,1H,Py); 7,90 dtr (J=7.5, 1 Hz,1H, Py); 7,45-7,55 m (4H,Ar); 7,35 dd (J=7.5, 4.5 Hz,1H,Py); 5,80 dd (J=5, 2.5 Hz,1H,H-16); 3,90-4,00 m (4H,Ketal); 3,30 ddbr (J=5, 7 Hz,1H,H-11); 1,96 s (3H,Propin); 0,61 s (3H,C-18)
G) 17-Chlor-17aβ-hydroxy-17aα-(1-propinyl)-11β-[4(3-pyridinyl)phenyl]-17a-homoestra-4,16-dien-3-on (2G)
   Analog zu Beispiel 1L) werden 925 mg 2F mit 2 ml 2 normaler wäßriger Salzsäure in 25 ml Aceton umgesetzt. Man erhält nach Chromatographie an Kieselgel mit einem Gemisch aus Hexan/Ethylacetat sowie Umkristallisation aus Diisopropylether 619 mg der Titelverbindung 2G als weiße Kristalle.
   ¹H-NMR (CDCl₃): δ= 8,87 sbr (1H, Py); 8,58 dbr (J=4.5 Hz, 1H, Py); 7,89 dtr (J=7.5, 1 Hz, 1H, Py); 7,37 dd (J=7.5, 4.5 Hz, 1H, Py); 7,58 d (J=10 Hz, 2H, Ar); 7,52 d (J=10 Hz, 2H, Ar); 5,88 sbr (1H, H-4); 5,80 dd (J=5, 2.5 Hz,1H,H-16); 3,51 ddbr (J=7, 5 Hz, 1H, H-11); 2,36 m (1H, H-10); 1,93 s (3H, Me); 0,68 s (3H, H-18)
   [α]²⁰_{D}=-67,4° (CHCl₃; c=0,525)
   Fp= 195°C

### Beispiel 3

### Herstellung von 11β-(4-Acetylphenyl)-17-chlor-17aβ-hydroxy-17aα-(1-propinyl)-17a-homoestra-4,16-dien-3-on (3B)

A) 17-Chlor-3,3-[1,2-ethandiylbis(oxy)]-11β-[4-(1-ethoxyethenyl)phenyl]-17aα-(1-propinyl)-17a-homo-5α-estr-16-en-5,17aβ-diol (3A)
   500 mg 2E werden in 6 ml Dioxan gelöst. Man addiert unter Argon 45 mg Tetrakis(triphenylphosphin)palladium, 67 mg Lithiumchlorid sowie 0,09 ml Pyridin und kocht 2 Stunden unter Rückfluß. Anschließend wird die Reaktionslösung über Celite flitriert, mit Ethylacetat verdünnt, mit gesättigter wäßriger Natriumchloridlösung gewaschen, über Natriumsulfat getrokknet, filtriert und am Vakuum eingeengt. Man erhält 435 mg 3A als weißen Schaum. Das Rohprodukt wird ohne weitere Reinigung in die Folgestufe eingesetzt.
B) 11β-(4-Acetylphenyl)-17-chlor-17aβ-hydroxy-17aα-(1-propinyl)-17a-homoestra-4,16-dien-3-on (3B)
   435 mg 3A und 1,1 ml 2 normale wäßrige Salzsäure werden in 30 ml Aceton analog zu Beispiel 1L) umgesetzt. Man erhält nach Säulenchromatographie an Kieselgel mit einem Gemisch aus Hexan/Ethylacetat 275 mg der Titelverbindung 3B als weißen Schaum.
   ¹H-NMR (CDCl₃): δ= 7,90 d (J=8 Hz,2H,Ar); 7,53 d (J=8 Hz,2H,Ar); 5,87 sbr (1H,H-4); 5,80 dd (J=5, 2.5 Hz,1H,H-16); 3,50 ddbr (J=5, 7 Hz,1H,H-11); 2,60 s (3H,Acetyl); 1,90 s (3H,Propin); 0,62 s (3H,C-18)
   [α]²⁰_{D}=-88,7° (CHCl₃; c=0,505)

### Beispiel 4

### Herstellung von 17-Chlor-11β-[4-(3-furanyl)phenyl]-17aβ-hydroxy-17aα-(1-propinyl)-17a-homoestra-4,16-dien-3-on (4B)

A) 17-Chlor-3,3-[1,2-ethandiylbis(oxy)]-11β-[4-(3-furanyl)phenyl]-17aα-(1-propinyl)-17a-homo-5α-estr-16-en-5,17aβ-diol (4A)
   Wie unter Beispiel 3A) beschrieben werden 1,76 g 2E, 1,58 g (3-Furanyl)tributylstannan (Herstellung siehe *Synthesis* 898 (1985)), 155 mg Tetrakis(triphenylphosphin)palladium, 231 mg Lithiumchlorid sowie 0,29 ml Pyridin in 21 ml Dioxan umgesetzt. Man erhält nach Säulenchromatographie an Kieselgel mit einem Gemisch aus Hexan/Ethylacetat 1,23 g 4A als weißen Schaum.
   ¹H-NMR (CDCl₃): δ= 7,72 dbr (J=1.3 Hz,1H,Fu-2); 7,45-7,50 m (3H, Ar+Fu-5); 6,70 dbr (J=1.8 Hz,1H,Fu-4); 5,79 dd (J=5, 2.5 Hz,1H,H-16); 3,90-4,00 m (4H,Ketal); 3,29 ddbr (J=5, 7 Hz,1H,H-11); 1,94 s (3H,Propin); 0,62 s (3H,C-18)
B) 17-Chlor-11β-[4-(3-furanyl)phenyl]-17aβ-hydroxy-17aα-(1-propinyl)-17a-homoestra-4,16-dien-3-on (4B)
   Wie unter Beispiel 1L) beschrieben werden 1,23 g 4A und 2,8 ml 2 molare wäßrige Salzsäure in 95 ml Aceton umgesetzt. Man erhält nach Säulenchromatographie an Kieselgel mit einem Gemisch aus Hexan/Ethylacetat 820 mg der Titelverbindung 4B als weißen Schaum.
   ¹H-NMR (CDCl₃): δ= 7,72 dbr (J=1.3 Hz,1H,Fu-2); 7,47 dd (J=1.8, 1.3 Hz,1H,Fu-5); 7,38-7,45 m (4H,Ar); 6,70 dbr (J=1.8 Hz,1H,Fu-4); 5,87 sbr (1H,H-4); 5,80 dd (J=5, 2.5 Hz,1H,H-16); 3,44 ddbr (J=5, 7Hz,1H,H-11); 1,93 s (3H,Propin); 0,70 s (3H,C-18)
   Fp: 158,5°C (Zersetzung)
   [α]²⁰_{D}=-67,8° (CHCl₃; c=0,510)

### Beispiel 5

### Herstellung von 17-Chlor-11β-[4-(3-furanyl)phenyl]-17aβ-hydroxy-17aα-methyl-17a-homoestra-4,16-dien-3-on (5C)

A) 17-Chlor-3,3-[1,2-ethandiylbis(oxy)]-17aα-methyl-11β-[4-[[(trifluormethyl)sulfonyl]oxy]phenyl]-17a-homo-5α-estr-16-en-5,17aβ-diol (5A)
   3,6 ml einer 1,6 molaren Lösung von Methyllithium in Diethylether werden mit 5 ml absolutem Tetrahydrofuran versetzt. Man kühlt die Lösung auf 0°C und tropft 687 mg der unter Beispiel 2D) hergestellten Verbindung, gelöst in 6 ml absolutem Tetrahydrofuran, hinzu. Man rührt eine Stunde bei 0°C nach, gießt dann die Reaktionslösung vorsichtig auf Wasser und extrahiert mit Ethylacetat. Die organische Phase wird mit gesättigter wäßriger Natriumchloridlösung gewaschen, über Natriumsulfat getrocknet, filtriert und am Vakuum eingeengt. Das Rohprodukt wird durch Säulenchromatographie an Kieselgel mit einem Gemisch aus Hexan/Ethylacetat gereinigt. Man erhält 604 mg 5A als weißen Schaum.
   ¹H-NMR (CDCl₃): δ= 7,50 d (J=8 Hz,2H,Ar); 7,18 d (J=8 Hz,2H,Ar); 5,77 dd (J=5, 2.5 Hz, 1H,H-16); 3,90-4,00 m (4H,Ketal); 3,30 ddbr (J=5, 7 Hz,1H,H-11); 1,30 s (3H,C-20); 0,58 s (3H,C-18)
B) 17-Chlor-3,3-[1,2-ethandiylbis(oxy)]-11β-[4-(3-furanyl)phenyl]-17aα-methyl-17a-homo-5α-estr-16-en-5,17aβ-diol (5B)
   Analog zu Beispiel 3A) werden 604 mg 5A, 414 mg (3-Furanyl)tributylstannan, 41 mg Tetrakis(triphenylphosphin)palladium, 61 mg Lithiumchlorid sowie 0,08 ml Pyridin in 10 ml Dioxan umgesetzt. Man erhält nach Säulenchromatographie an Kieselgel mit einem Gemisch aus Hexan/Ethylacetat 363 mg 5B als weißen Schaum.
   ¹H-NMR (CDCl₃): δ= 7,72 dbr (J=1.3 Hz,1H,Fu-2); 7,48 dd (J=1.8, 1.3 Hz,1H,Fu-5); 7,42 d (J=8 Hz,2H,Ar); 7,39 d (J=8 Hz,2H,Ar); 6,70 dbr (J=1.8 Hz,1H,Fu-4); 5,73 dd (J=5, 2.5 Hz,1H,H-16); 3,90-4,00 m (4H,Ketal); 3,28 ddbr (J=7, 5 Hz,1H,H-11); 1,31 s (3H,C-20); 0,62 s (3H, C-18)
C) 17-Chlor-11β-[4-(3-furanyl)phenyl]-17aβ-hydroxy-17aα-methyl-17a-homoestra-4,16-dien-3-on (5C)
   Wie unter Beispiel 1L) beschrieben werden 363 mg 5B und 0,88 ml 2 normale wäßrige Salzsäure in 35 ml Aceton umgesetzt. Man erhält nach Säulenchromatographie und Umkristallisation aus Diisopropylether 240 mg der Titelverbindung 5C als weiße Kristalle.
   ¹H-NMR (CDCl₃): δ= 7,73 dbr (J=1.3 Hz,1H,Fu-2); 7,49 dd (J=1.8, 1.3 Hz,1H,Fu-5); 7,45 d (J=8 Hz,2H,Ar); 7,40 d (J=8 Hz,2H,Ar); 6,70 dbr (J=1.8 Hz,1H,Fu-4); 5,89 sbr (1H,H-4); 5,78 dd (J=5, 2.5 Hz,1H,H-16); 3,45 ddbr (J=5, 7 Hz,1H,H-11); 1,33 s (3H,C-20); 0,71 s (3H,C-18)
   Fp: 221,3°C (Zersetzung)
   [α]²⁰_{D}= +106,0° (CHCl₃; c=0,530)

### Beispiel 6

### Herstellung von 17aβ-Hydroxy-11β-(4-methoxyphenyl)-17aα-(1-propinyl)-17a-homoestra-4,16-dien-3-on (6E)

A) 16β,17β-Dihydro-3,3-[1,2-ethandiylbis(oxy)]-11β-(4-methoxyphenyl)-17β-[(trimethylsilyl)oxy)-3'H-cyclopropa[16,17]-5α-estran-5-ol (6A)
   Darstellung des Zink-Silber-Paares: Zu einer Lösung von 12 mg Silber(I)acetat in 12 ml Eisessig werden bei 100°C 2 g Zinkpulver (aktiviert mit 2 molarer Salzsäure) gegeben. Man rührt 30 Sekunden bei 100°C nach, dekantiert den Eisessig ab und wäscht den erhaltenen Feststoff einmal mit Eisessig sowie fünfmal mit absolutem Diethylether. Danach wird sechs Stunden am Hochvakuum getrocknet.
   Cyclopropanierung: 655 mg des Zink-Silber-Reagenzes werden mit 2,5 ml absolutem Diethylether versetzt. Anschließend addiert man 0,64 ml Diiodmethan und erwärmt leicht im Ölbad bis eine exotherme Reaktion beobachtet wird. Man entfernt die Heizquelle und beobachtet, ohne daß weitere Wärme zugeführt wird, ein leichtes Rückflußkochen. Man läßt nachrühren bis keine Reaktion mehr zu beobachten ist (ca. 25 Minuten), verdünnt dann mit 8,5 ml absolutem Diethylether und läßt weitere 30 Minuten bei nachrühren. Anschließend werden 513 mg 1H, gelöst in 5 ml absolutem Diethylether addiert. Man kocht 30 Minuten unter Rückfluß, läßt die Mischung auf Raumtemperatur kommen, addiert 0,93 ml Pyridin, filtriert über Celite und engt am Vakuum ein. Das erhaltene Rohprodukt wird durch Säulenchromatographie an Kieselgel mit einem Gemisch aus Hexan/Ethylacetat gereinigt. Man erhält 211 mg 6A als weißen Schaum.
   ¹H-NMR (CDCl₃): δ= 7,28 d (J=8 Hz,2H,Ar); 6,79 d (J=8 Hz,2H,Ar); 3,90-4,00 m (4H,Ketal); 3,80 s (3H,OMe); 3,18 ddbr (J=5, 7 Hz,1H,H-11); 1,00 m (1H,Dreiring); 0,85 m (1H, Dreiring); 0,74 s (3H,C-18); 0,70 m (1H,Dreiring); 0,10 m (9H,SiMe₃)
B) 16ξ-Chlor-3,3-[1,2-ethandiylbis(oxy)]-5-hydroxy-11β-(4-methoxyphenyl)-17a-homo-5α-estran-17a-on (6B)
   Zu 2,8 ml absolutem Dimethylformamid werden unter Argon bei 0°C 788 mg wasserfreies Eisen(III)chlorid addiert. Anschließend tropft man eine Lösung von 406 mg 6A in 6 ml Dichlormethan und 0,4 ml Pyridin hinzu und läßt 3 Stunden nachrühren wobei die Temperatur langsam auf Raumtemperatur steigt. Danach wird die Reaktionsmischung auf gesättigte wässrige Natriumhydrogencarbonatlösung gegossen. Man extrahiert mit Ethylacetat, wäscht die organische Phase mit gesättigter wäßriger Ammoniumchloridlösung sowie mit gesättigter wäßriger Natriumchloridlösung, trocknet über Natriumsulfat, filtriert und eng am Vakuum ein. Man erhält 370 mg 6B. Das Rohprodukt wird ohne Reinigung in die Folgestufe eingesetzt.
C) 3,3-[1,2-Ethandiylbis(oxy)]-5-hydroxy-11β-(4-methoxyphenyl)-17a-homo-5α-estr-16-en-17a-on (6C)
   Eine Mischung aus 370 mg 6B und 1,26 g Natriumacetat in 9 ml Ethanol wird 3 Stunden unter Rückfluß gekocht. Anschließend wird die Reaktionsmischung mit Ethylacetat verdünnt, mit gesättigter wäßriger Natriumchloridlösung gewaschen, über Natriumsulfat getrocknet, filtriert und am Vakuum eingeengt. Man erhält nach Säulenchromatographie an Kieselgel mit einem Gemisch aus Hexan/Ethylacetat 244 mg 6C als weißen Schaum.
   ¹H-NMR (CDCl₃): δ= 7,28-7,35 m (3H,H-16+Ar); 6,77 d (J=8 Hz,2H,Ar); 5,82 ddd (J=10, 1, 1 Hz,1H,H-17); 3,90-4,00 m (4H,Ketal); 3,80 s (3H,OMe); 3,56 ddbr (J=5, 7 Hz,1H,H-11); 0,49 s (3H,C-18)
D) 3,3-[1,2-Ethandiylbis(oxy)]-5-hydroxy-11β-(4-methoxyphenyl)-17aα-(1-propinyl)-17a-homo-5α-estr-16-en-5,17aβ-diol (6D)
   Analog zu Beispiel 1K) werden aus 244 mg 6C, 2,98 ml einer 1,6 molaren Lösung von Butyllithium und Propingas in 15 ml absolutem Tetrahydrofuran nach Säulenchromatographie an Kieselgel mit einem Gemisch aus Hexan/Ethylacetat 212 mg 6D als weißer Schaum erhalten.
   ¹H-NMR (CDCl₃): δ= 7,31 d (J=8 Hz,2H,Ar); 6,78 d (J=8 Hz,2H,Ar); 5,63 ddd (J=10, 1, 1 Hz,1H,H-16); 5,50 dbr (J=10 Hz,1H,H-17); 3,90-4,00 m (4H,Ketal); 3,80 s (3H,OMe); 3,22 ddbr (J=5, 7 Hz,1H,H-11); 1,92 s (3H,Propin); 0,57 s (3H,C-18)
E) 17aβ-Hydroxy-11β-(4-methoxyphenyl)-17aα-(1-propinyl)-17a-homoestra-4,16-dien-3-on (6E)
   Wie unter Beispiel 1L) beschrieben werden aus 212 mg 6D und 0,5 ml 2 normaler wäßriger Salzsäure in 9 ml Aceton nach Säulenchromatographie an Kieselgel mit einem Gemisch aus Hexan/Ethylacetat 120 mg der Titelverbindung 6E als weißer Schaum erhalten.
   ¹H-NMR (CDCl₃): δ= 7,37 d (J=8 Hz,2H,Ar); 6,82 d (J=8 Hz,2H,Ar); 5,87 sbr (1H,H-4); 5,67 ddd (J=10, 1, 1 Hz,1H,H-16); 5,52 dbr (J=10 Hz,1H,H-17); 3,80 s (3H, OMe); 3,40 ddbr (J=5, 7 Hz,1H,H-11); 1,92 s (3H,Propin); 0,64 s (3H,C-18)

### Beispiel 7

### Herstellung von 17aβ-Hydroxy-11β-(4-methoxyphenyl)-17aα-methyl-17a-homoestra-4,16-dien-3-on (7C)

A) 17-Chlor-3,3-[1,2-ethandiylbis(oxy)]-11β-(4-methoxyphenyl)-17aα-methyl-17a-homo-5α-estra-5,17aβ-diol (7A)
   Analog zu Beispiel 5A) werden 490 mg 1I und 3,15 ml einer 1,6 molaren Lösung von Methyllithium in Diethylether in 18 ml absolutem Tetrahydrofuran umgesetzt. Man erhält nach Säulenchromatographie an Kieselgel mit einem Gemisch aus Hexan/Ethylacetat 405 mg 7A als weißen Schaum.
   ¹H-NMR (CDCl₃): δ= 7,31 d (J=8 Hz,2H,Ar); 6,79 d (J=8 Hz,2H,Ar); 5,75 dd (J=5, 1 Hz,1H, H-16); 3,90-4,00 m (4H,Ketal); 3,80 s (3H,OMe); 3,21 ddbr (J=5, 7 Hz,1H,H-11); 1,31 s (3H, C-20); 0,61 s (3H,C-18)
B) 3,3-[1,2-Ethandiylbis(oxy)]-11β-(4-methoxyphenyl)-17aα-methyl-17a-homo-5α-estr-16-en-5,17β-diol (7B)
   Zu 7 ml Ammoniak werden bei -78°C 20 mg Lithium gegeben. Nachdem die charakteristische Blaufärbung aufgetreten ist, werden 404 mg 7A, gelöst in 4 ml absolutem Tetrahydrofuran, zügig addiert, wobei eine Entfärbung der Reaktionsmischung beobachtet wird. Sobald erneute Blaufärbung beobachtet wird, addiert man vorsichtig Wasser und läßt den Ammoniak abziehen. Anschließend wird mit Ethylacetat extrahiert. Man wäscht die organische Phase mit gesättigter wäßriger Ammoniumchloridlösung sowie mit gesättigter wäßriger Natriumchloridlösung, trocknet über Natriumsulfat, filtriert und engt am Vakuum ein. Das Rohprodukt wird durch Säulenchromatographie an Kieselgel mit einem Gemisch aus Hexan/Ethylacetat gereinigt. Man erhält 347 mg 7B als weißen Schaum.
   ¹H-NMR (CDCl₃): δ= 7,32 d (J=8 Hz,2H,Ar); 6,28 d (J=8 Hz,2H,Ar); 5,55 ddd (J=10, 1, 1 Hz,1H,H-16); 5,34 dbr (J=10 Hz,1H,H-17); 3,90-4,00 m (4H,Ketal); 3,80 s (3H, OMe); 3,21 ddbr (J=5, 7 Hz,1H,H-11); 1,25 s (3H,C-20); 0,59 s (3H,C-18)
C) 17aβ-Hydroxy-11β-(4-methoxyphenyl)-17aα-methyl-17a-homoestra-4,16-dien-3-on (7C)
   Wie unter Beispiel 1L) beschrieben werden aus 347 mg 7B und 1 ml 2 normaler wäßriger Salzsäure in 20 ml Aceton nach Säulenchromatographie an Kieselgel mit einem Gemisch aus Hexan/Ethylacetat 226 mg der Titelverbindung 7C als weißer Schaum erhalten.
   ¹H-NMR (CDCl₃): δ= 7,36 d (J=8 Hz,2H,Ar); 6,82 d (J=8 Hz,2H,Ar); 5,86 sbr (1H,H-4); 5,58 ddd (J=10, 1, 1 Hz,1H,H-16); 5,39 dbr (J=10 Hz,1H,H-17); 3,80 s (3H,OMe); 3,45 ddbr (J=5, 7 Hz,1H,H-11); 1,25 s (3H,C-20); 0,66 s (3H,C-18)

### Beispiel 8

### Herstellung von 17aβ-Hydroxy-11β-(4-methoxyphenyl)-17aα-(1-propinyl)-17a-homoestr-4-en-3-on (8C)

A) 3,3-[1,2-Ethandiylbis(oxy)]-5-hydroxy-11β-(4-methoxyphenyl)-17a-homo-5α-estran-17a-on (8A)
   244 mg 1I, 5 mg Azoisobutyronitril und 0,4 ml Tributylzinnhydrid werden in 20 ml absolutem Toluol 1,5 Stunden unter Rückfluß gekocht. Nach dem Abkühlen wird am Vakuum eingeengt und der Rückstand durch Säulenchromatographie an Kieselgel mit einem Gemisch aus Hexan/Ethylacetat gereinigt. Man erhält 160 mg 8A als weißen Schaum.
   ¹H-NMR (CDCl₃): δ= 7,31 d (J=8 Hz,2H,Ar); 6,29 d (J=8 Hz,2H,Ar); 3,90-4,00 m (4H,Ketal); 3,79 s (3H,OMe); 3,20 ddbr (J=5, 7 Hz,1H,H-11); 0,80 s (3H,C-18)
B) 3,3-[1,2-Ethandiylbis(oxy)]-11β-(4-methoxyphenyl)-17aα-(1-propinyl)-17a-homo-5α-estra-5,17aβ-diol (8B)
   Analog zu Beispiel 1K) werden aus 338 mg 8A, 4,65 ml einer 1,6 molaren Lösung von Butyllithium in Hexan und Propingas in 20 ml absolutem Tetrahydrofuran nach Säulenchromatographie an Kieselgel mit einem Gemisch aus Hexan/Ethylacetat 246 mg 8B als weißer Schaum erhalten.
   ¹H-NMR (CDCl₃): δ= 7,30 d (J=8 Hz,2H,Ar), 6,29 d (J=8 Hz,2H,Ar); 3,90-4,00 m (4H,Ketal); 3,80 s (3H,OMe); 3,19 ddbr (J=5, 7 Hz,1H,H-11); 1,93 s (2H,Propin); 0,60 s (3H,C-18)
C) 17aβ-Hydroxy-11β-(4-methoxyphenyl)-17aα-(1-propinyl)-17a-homoestr-4-en-3-on (8C)
   Analog zu Beispiel 1L) werden aus 246 mg 8B und 0,6 ml 2 normaler wäßriger Salzsäure in 10 ml Aceton nach Säulenchromatographie an Kieselgel mit einem Gemisch aus Hexan/ Ethylacetat 184 mg der Titelverbindung 8C als weißer Schaum erhalten.
   ¹H-NMR (CDCl₃): δ= 7,33 d (J=8 Hz,2H,Ar); 6,83 d (J=8 Hz,2H,Ar); 5,85 sbr (1H,H-4); 3,80 s (3H,OMe); 3,35 ddbr (J=5, 7 Hz,1H,H-11); 1,90 s (3H,Propin); 0,67 s (3H,C-20)

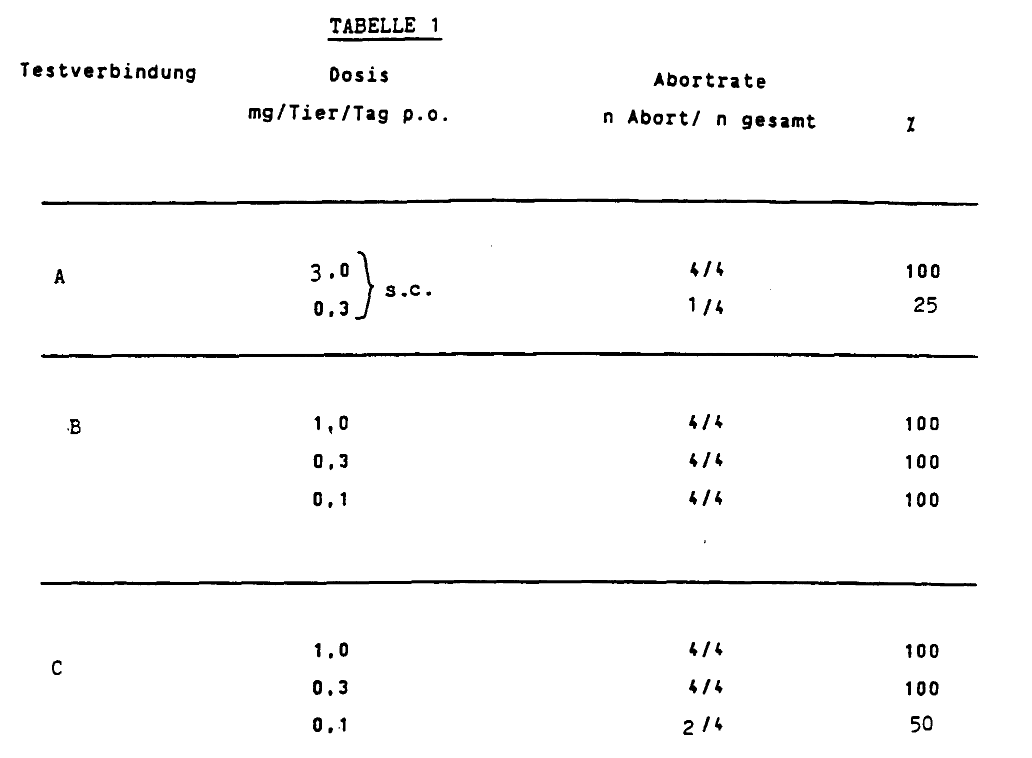

## Patentansprüche

1. Verbindungen der allgemeinen Formel I 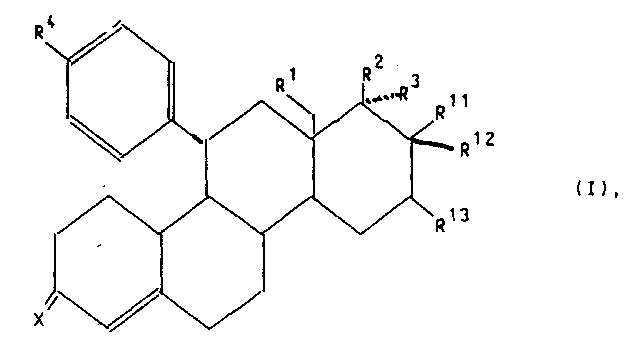 worin
X für ein Sauerstoffatom, die Hydroxyiminogruppierung >N OH oder zwei Wasserstoffatome,
R¹ für ein Wasserstoffatom oder eine Methylgruppe,
R² für eine Hydroxygruppe, eine C₁-C₁₀-Alkoxy- oder C₁-C₁₀-Acyloxygruppe,
R³ für ein Wasserstoffatom, die Gruppierung -(CH₂)ₙCH₂Z, wobei n 0, 1, 2, 3, 4 oder 5 ist, Z ein Wasserstoffatom, die Cyanogruppe oder den Rest-OR⁵ mit R⁵=H, C₁-C₁₀-Alkyl oder C₁-C₁₀-Acyl bedeuten, die Gruppierung -(CH₂)ₘC≡C-Y, wobei m 0, 1 oder 2 und Y ein Wasserstoff-, Fluor-, Chlor-, Brom- oder Jod-Atom, einen C₁-C₁₀-Hydroxyalkyl-, C₁-C₁₀-Alkoxyalkyl-, C₁-C₁₀-Acyloxyalkylrest, oder wenn m = 0 ist, zusätzlich eine Methylgruppe bedeuten, die Gruppierung -(CH₂)ₚ-CH=CH-(CH₂)ₖCH₂R⁶, wobei p 0 oder 1 und k 0, 1 oder 2 und R⁴ ein Wasserstoffatom, eine Hydroxygruppe, einen C₁-C₄-Alkoxy- oder C₁-C₄-Acyloxyrest bedeuten,
oder aber R² und R³ gemeinsam für einen Rest der Formel 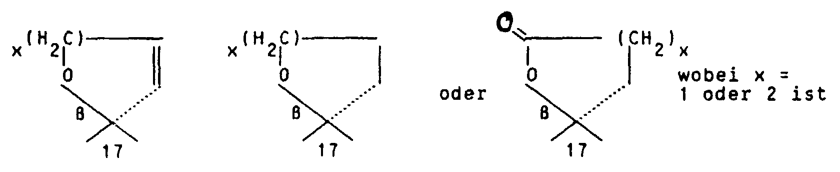 R⁴ für ein Wasserstoffatom, eine Cyanogruppe, ein Chlor-, Fluor-, Brom-, Jodatom, für eine Trialkylsilyl-, Trialkylstannylgruppe, für einen geradkettigen oder verzweigten, gesättigten oder ungesättigten C₁-C₈-Alkyl-, -Acyl-oder Alkoxyalkylrest, für eine 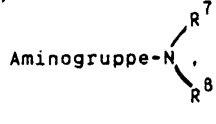 in welcher R⁷ und R⁸ unabhängig voneinander ein Wasserstoffatom oder eine C₁-C₄-Alkylgruppe bedeuten, oder für ein entsprechendes Aminoxid 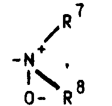 oder für die Gruppierungen -OR⁹ oder -S(O)ᵢR⁹ mit i = 0, 1 oder 2, in welchen R⁹ ein Wasserstoffatom, eine Methyl-, Ethyl-, Propyl-, Isopropyl-, Methoxyphenyl-, Allyl-oder eine 2-Dimethylaminoethylgruppe bedeuten, oder für einen Heteroarylrest der Formel Iα 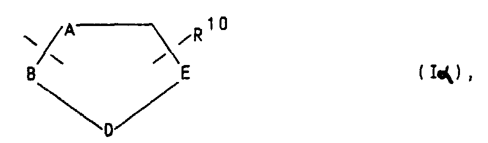 in welchem A ein Stickstoff-, Sauerstoff- oder Schwefelatom, -B-D-E-die Elementenfolge -C-C-C-, -N-C-C- oder -C-N-C- und R¹⁰ ein Wasserstoffatom, eine Cyanogruppe, ein Chlor-, Fluor-, Brom-, Jodatom, eine Trialkylsilyl-, Trialkylstannylgruppe, einen geradkettigen oder verzweigten, gesättigten oder ungesättigten C₁-C₈-Alkyl-, -Acyl-oder Alkoxyalkylrest, für eine 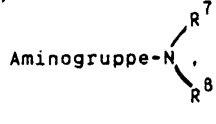 in welcher R⁷ und R⁸ unabhängig voneinander ein Wasserstoffatom oder eine C₁-C₄-Alkylgruppe bedeuten, oder ein entsprechendes Aminoxid 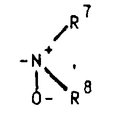 oder die Gruppierung -OR⁹ oder -S(O)ᵢR⁹ mit i = 0, 1 oder 2, in welchen R⁹ ein Wasserstoffatom, eine Methyl-, Ethyl-, Propyl-, Isopropyl-, Methoxyphenyl-, Allyl-oder eine 2-Dimethylaminoethylgruppe bedeuten, symbolisieren,
oder für einen Heteroarylrest der Formel Iβ 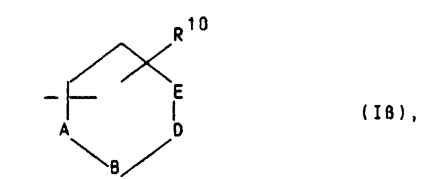 in welchem A ein Stickstoffatom und -B-D-E- die Elementenfolge -C-C-C-, -N-C-C-, -C-N-C- oder -C-C-N- bedeuten unbd R¹⁰ die bereits angegebene Bedeutung hat,
oder für einen Phenylrest der Formel Iγ 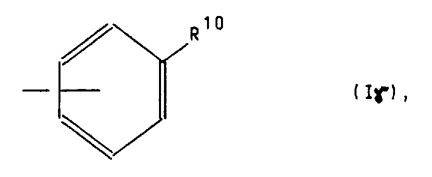 worin R¹⁰ die bereits angegebene Bedeutung hat,
R¹¹ für ein Fluor-, Chlor- oder Bromatom, wobei dann R¹² und R¹³ gemeinsam eine zusätzliche Bindung bedeuten oder
R¹¹ für einen gerad- oder verzweigtkettigen C₁-C₄-Alkylrest oder ein Wasserstoffatom, wobei dann R¹² und R¹³ je ein Wasserstoffatom oder gemeinsam eine zusätzliche Bindung bedeuten,
stehen.

2. Verbindungen nach Anspruch 1, nämlich
17aβ-Hydroxy-11β-(4-methoxyphenyl)-17aα-(1-propinyl)-17a-homoestra-4,16-dien-3-on
17aβ-Hydroxy-11β-(4-methoxyphenyl)-17-aα-methyl-17a-homoestra-4,16-dien-3-on
11β[4-(3-Furanyl)phenyl]-17aβ-hydroxy-17aα-(1-propinyl)-17a-homoestra-4,16-dien-3-on
11β-(4-Acetylphenyl)-17aβ-hydroxy-17aα-(1-propinyl)-17a-homoestra-4,16-dien-3-on
17aβ-Hydroxy-17aα-(3-hydroxypropyl)-11β-[4-(3-pyridinyl)phenyl]-17a-homoestra-4,16-dien-3-on
(Z)-4'-[17aβ-Hydroxy-17aα-(3-hydroxy-1-propenyl)-3-oxo-17a-homo-4,16-dien-11β-yl]-[1,1'-biphenyl]-4-carbonitril
11β-[4-(3-Furanyl)phenyl]-17aβ-hydroxy-17aα-methyl-17a-homoestra-4,16-dien-3-on
11β-(4-Acetylphenyl)-17aβ-hydroxy-17aα-(3-hydroxypropyl)-17a-homoestra-4-en-3-on
11β-(4-Acetylphenyl)-4',5'-dihydrospiro[17a-homoestra-4-en-17aβ,2'(3H)-furan]-3-on
(Z)-4'-[17aβ-Hydroxy-17aα-(3-hydroxy-1-propenyl)-3-oxo-17a-homoestr-4-en-11β-yl]-[1,1'-biphenyl]-4-carbonitril
17-Chlor-11β-(4-methoxyphenyl)-17aβ-hydroxy-17aα-(1-propinyl)-17a-homoestra-4,16-dien-3-on
17-Chlor-17aβ-hydroxy-17aα-(1-propinyl)-11β-[4-(3-pyridinyl)phenyl]-17a-homoestra-4,16-dien-3-on
11β-(4-Acetylphenyl)-17-chlor-17aβ-hydroxy-17aα-(1-propinyl)-17a-homoestra-4,16-dien-3-on
17-Chlor-11β-[4-(3-furanyl)phenyl]-17aβ-hydroxy-17aα-(1-propinyl)-17a-homoestra-4,16-dien-3-on
17-Chlor-11β-[4-(3-furanyl)phenyl]-17aβ-hydroxy-17aα-methyl-17a-homoestra-4,16-dien-3-on
4'-[17-Chlor-17aβ-hydroxy-17aα-methyl-3-oxo-17a-homoestra-4,16-dien-11β-yl][1,1'-biphenyl]4-carbonitril
(Z)-11β-(4-Acetylphenyl)-17-chlor-17aβ-hydroxy-17aα-(3-hydroxy-1-propenyl)-17a-homoestra-4,16 dien-3-on
11β-(4-Acetylphenyl)-17-chlor-17aβ-hydroxy-17aα-(3-hydroxypropyl)-17a-homoestra4,16-dien-3-on
17-Chlor-11β-[4-(3-furanyl)phenyl]-17aβ-hydroxy-3-oxo-17a-homoestra-4,16-dien-17aα-acetonitril
11β-(4-Acetylphenyl)-17-fluor-17aβ-hydroxy-17aα-methyl-17a-homoestra-4,16-dien-3-on
11β-(4-Acetylphenyl)-17-fluor-17aβ-hydroxy-17aα-(3-hydroxypropyl)-17a-homoestra-4,16-dien-3-on
11β-(4-Acetylphenyl)-17-fluor-17aβ-hydroxy-17aα-(1-propinyl)-17a-homoestra-4,16-dien-3-on
17aβ-Hydroxy-11β-(4-methoxyphenyl)-17aα-(1-propinyl)-17a-homoestr-4-en-3-on.

3. Zwischenprodukte der allgemeinen Formel F 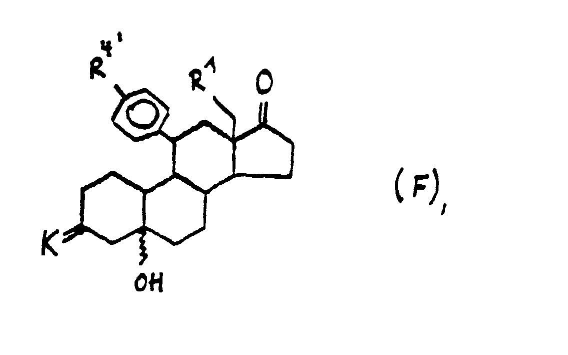 worin
K für die 1,2-Ethandiylbis(oxy)-, 1,3-Propandiylbis(oxy)-, 1,3-Propan-(2,2-dimethyl)-diylbis(oxy)gruppe oder für ein anderes Sauerstoffketal
R¹ für ein Wasserstoffatom oder eine Methylgruppe und
R^{4'} für einen der Reste R⁴ wie bereits in Anspruch 1 angegeben, stehen.

4. Pharmazeutische Präparate, enthaltend mindestens eine Verbindung gemäß Anspruch 1 oder 2 sowie einen pharmazeutischen Träger.

5. Verwendung der Verbindungen gemäß Anspruch 1 oder 2 zur Herstellung von Arzneimitteln.

6. Verfahren zur Herstellung von Verbindungen der allgemeinen Formel I 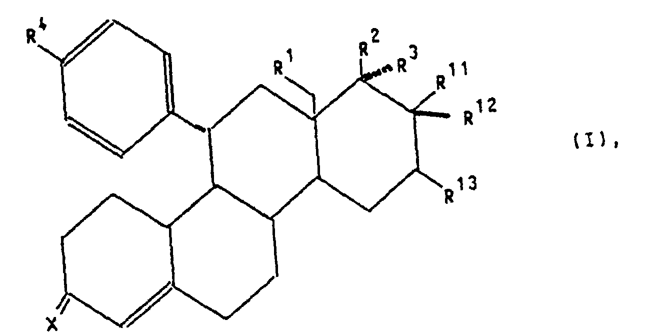 worin X, R¹, R², R³, R⁴, R¹¹, R¹² und R¹³ die in Anspruch 1 angegebene Bedeutung haben, dadurch gekennzeichnet, daß eine Verbindung der allgemeinen Formel J oder K 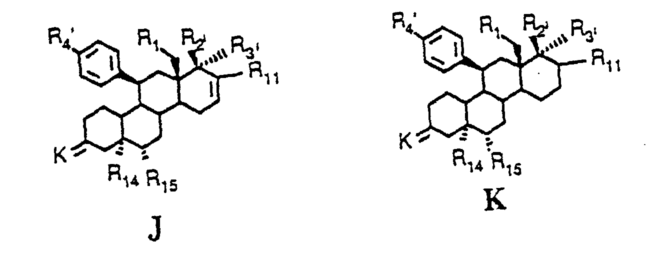 worin K eine als Ketal geschützte Ketogruppe ist, R¹ und R¹¹ die gleiche Bedeutung wie in Formel I sowie R^{2'}, R^{3'} und R^{4'} unter Ausschluß des Cyanid restes für R⁴ die gleiche Bedeutung wie R², R³ bzw. R⁴ in Formel I haben, wobei vorhandene Hydroxy-, Mercapto-, Amino-, Oxo- und/oder terminale Acetylengruppen gegebenenfalls geschützt sind, sowie R¹⁴ eine Hydroxygruppe und R¹⁵ ein Wasserstoff bedeuten, der Einwirkung eines sauren Agens, das zur Freisetzung der 3-Oxygruppe sowie der anderen geschützten Gruppen und zur Wasserabspaltung unter Ausbildung der 4(5)-Doppelbindung befähigt ist, unterworfen und anschließend gewünschtenfalls in R² und/oder R³ und/oder R⁴ vorhandene Hydroxy-, Mercapto-und/oder Aminogruppe(n) alkyliert bzw. acyliert, gewünschtenfalls ein Cyanidrest in den 11β-Arylsubstituenten eingeführt, gewünschtenfalls die in R⁴ gegebenenfalls enthaltene Amino- oder Sulfidgruppe oxidiert, gewünschtenfalls mit Hydroxylamin-hydrochlorid zum Produkt der allgemeinen Formel I mit X in der Bedeutung der Hydroxyiminogruppierung 〉N∼OH umgesetzt oder die 3-Oxogruppe in ein Produkt der allgemeinen Formel I, worin X für 2 Wasserstoffatome steht, umgewandelt sowie gegebenenfalls ein pharmazeutisch verträgliches Säureadditionssalz hergestellt wird/werden.

## Claims

1. Compounds of the general formula I 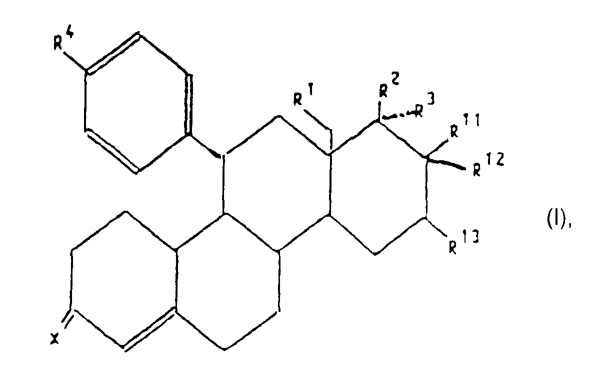 wherein
X is an oxygen atom, the hydroxyimino grouping >N∼OH or two hydrogen atoms.
R¹ is a hydrogen atom or a methyl group,
R² is a hydroxy group or a C₁-C₁₀-alkoxy or C₁-C₁₀-acyloxy group,
R³ is a hydrogen atom; the grouping -(CH₂)ₙCH₂Z wherein n is 0, 1, 2, 3, 4 or 5, Z is a hydrogen atom, the cyano group or the radical -OR⁵ wherein R⁵ = H, C₁-C₁₀-alkyl or C₁-C₁₀-acyl; the grouping -(CH₂)ₘC≡C-Y wherein m is 0, 1 or 2 and Y is a hydrogen, fluorine, chlorine, bromine or iodine atom, a C₁-C₁₀-hydroxyalkyl, C₁-C₁₀-alkoxyalkyl or C₁-C₁₀-acyloxyalkyl radical, or, when m = 0, additionally a methyl group; or the grouping -(CH₂)ₚ-CH=CH-(CH₂)ₖCH₂R⁶ wherein p is 0 or 1, and k is 0, 1 or 2 and R⁶ is a hydrogen atom, a hydroxy group or a C₁-C₄-alkoxy or C₁-C₄-acyloxy radical; or
R² and R³ together form a radical of the formula 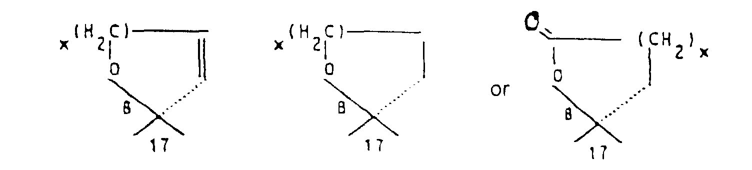 wherein x = 1 or 2,
R⁴ is a hydrogen atom, a cyano group, a chlorine, fluorine, bromine or iodine atom, a trialkylsilyl or trialkylstannyl group, a straight-chain or branched, saturated or unsaturated C₁-C₈-alkyl, -acyl or -alkoxyalkyl radical, an amino group 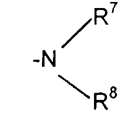 wherein R⁷ and R⁸ are each independently of the other a hydrogen atom or a C₁-C₄-alkyl group, or a corresponding amine oxide 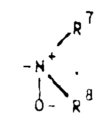 or the grouping -OR⁹ or -S(O)ᵢR⁹ wherein i = 0, 1 or 2 and R⁹ is a hydrogen atom or a methyl, ethyl, propyl, isopropyl, methoxyphenyl, allyl or 2-dimethylaminoethyl group, or a heteroaryl radical of the formula Iα 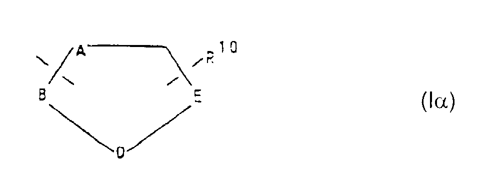 wherein A is a nitrogen, oxygen or sulphur atom, -B-D-E- is the element sequence -C-C-C-, -N-C-C- or -C-N-C- and R¹⁰ is a hydrogen atom, a cyano group, a chlorine, fluorine, bromine or iodine atom, a trialkylsilyl or trialkylstannyl group, a straight-chain or branched, saturated or unsaturated C₁-C₈-alkyl, -acyl or -alkoxyalkyl radical, an amino group 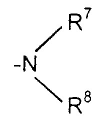 wherein R⁷ and R⁸ are each independently of the other a hydrogen atom or a C₁-C₄-alkyl group, or a corresponding amine oxide 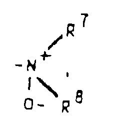 or the grouping -OR⁹ or -S(O)ᵢR⁹ wherein i = 0, 1 or 2 and R⁹ is a hydrogen atom or a methyl, ethyl, propyl, isopropyl, methoxyphenyl, allyl or 2-dimethylaminoethyl group,
or a heteroaryl radical of the formula Iβ 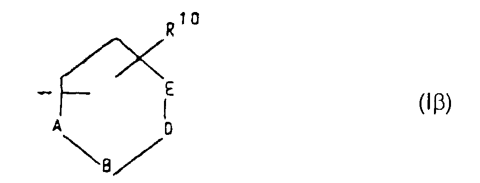 wherein A is a nitrogen atom and -B-D-E- is the element sequence -C-C-C-, -N-C-C-, -C-N-C- or -C-C-N- and R¹⁰ has the meaning given above,
or a phenyl radical of the formula Iγ 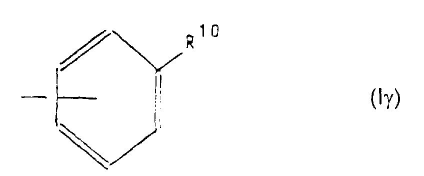
wherein
R¹⁰ has the meaning given above,
R¹¹ is a fluorine, chlorine or bromine atom, in which case R¹² and R¹³ together form an additional bond, or
R¹¹ is a straight-chain or branched C₁-C₄-alkyl radical or a hydrogen atom, in which case R¹² and R¹³ are each a hydrogen atom or together form an additional bond.

2. Compounds according to claim 1, namely
17aβ-hydroxy-11β-(4-methoxyphenyl)-17aα-(1-propynyl)-17a-homo-oestra-4,16-dien-3-one,
17aβ-hydroxy-11β-(4-methoxyphenyl)-17aα-methyl-17a-homo-oestra-4,16-dien-3-one,
11β-[4-(3-furanyl)phenyl]-17aβ-hydroxy-17aα-(1-propynyl)-17a-homo-oestra-4,16-dien-3-one,
11β-(4-acetylphenyl)-17aβ-hydroxy-17aα-(1-propynyl)-17a-homo-oestra-4,16-dien-3-one,
17aβ-hydroxy-17aα-(3-hydroxypropyl)-11β-[4-(3-pyridinyl)phenyl]-17a-homo-oestra-4,16-dien-3-one,
(Z)-4'-[17aβ-hydroxy-17aα-(3-hydroxy-1-propenyl)-3-oxo-17a-homo-oestra-4,16-dien-11β-yl][1,1'-biphenyl]-4-carbonitrile,
11β-[4-(3-furanyl)phenyl]-17aβ-hydroxy-17aα-methyl-17a-homo-oestra-4,16-dien-3-one,
11β-(4-acetylphenyl)-17aβ-hydroxy-17aα-(3-hydroxypropyl)-17a-homo-oestr-4-en-3-one,
11β-(4-acetylphenyl)-4',5'-dihydrospiro[17a-homo-oestr-4-ene-17aβ,2'(3H)-furan]-3-one,
(Z)-4'-[17aβ-hydroxy-17aα-(3-hydroxy-1-propenyl)-3-oxo-17a-homo-oestr-4-en-11β-yl]-[1,1'-biphenyl]-4-carbonitrile,
17-chloro-11β-(4-methoxyphenyl)-17aβ-hydroxy-17aα-(1-propynyl)-17a-homo-oestra-4,16-dien-3-one,
17-chloro-17aβ-hydroxy-17aα-(1-propynyl)-11β-[4-(3-pyridinyl)phenyl]-17a-homo-oestra-4,16-dien-3-one,
11β-(4-acetylphenyl)-17-chloro-17aβ-hydroxy-17aα-(1-propynyl)-17a-homo-oestra-4,16-dien-3-one,
17-chloro-11β-[4-(3-furanyl)phenyl]-17aβ-hydroxy-7aα-(1-propynyl)-17a-homo-oestra-4,16-dien-3-one,
17-chloro-11β-[4-(3-furanyl)phenyl]-17aβ-hydroxy-17aα-methyl-17a-homo-oestra-4,16-dien-3-one,
4'-[17-chloro-17aβ-hydroxy-17aα-methyl-3-oxo-17a-homo-oestra-4,16-dien-11β-yl]-[1,1'-biphenyl]-4-carbonitrile,
(Z)-11β-(4-acetylphenyl)-17-chloro-17aβ-hydroxy-7aα-(3-hydroxy-1-propenyl)-17a-homo-oestra-4, 16-dien-3-one,
11β-(4-acetylphenyl)-17-chloro-17aβ-hydroxy-17aα-(3-hydroxypropyl)-17a-homo-oestra-4, 16-dien-3-one,
17-chloro-11β-[4-(3-furanyl)phenyl]-17aβ-hydroxy-3-oxo-17a-homo-oestra-4,16-diene-17aα-acetonitrile,
11β-(4-acetylphenyl)-17-fluoro-17aβ-hydroxy-17aα-methyl-17a-homo-oestra-4,16-dien-3-one,
11β-(4-acetylphenyl)-17-fluoro-17aβ-hydroxy-17aα-(3-hydroxypropyl)-17a-homo-oestra-4,16-dien-3-one,
11β-(4-acetylphenyl)-17-fluoro-17aβ-hydroxy-17aα-(1-propynyl)-17a-homo-oestra-4,16-dien-3-one,
17aβ-hydroxy-11β-(4-methoxyphenyl)-17aα-(1-propynyl)-17a-homo-oestr-4-en-3-one.

3. Intermediates of the general formula F 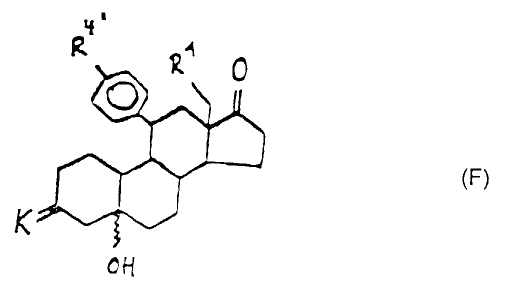 wherein
K is a 1,2-ethanediylbis(oxy), 1,3-propanediylbis(oxy) or 1,3-propane-(2,2-dimethyl)-diylbis(oxy) group or a different oxygen ketal,
R¹ is a hydrogen atom or a methyl group and
R^{4'} is one of the radicals R⁴ as already indicated in claim 1.

4. Pharmaceutical preparations, comprising at least one compound according to claim 1 or 2 and a pharmaceutical carrier.

5. Use of the compounds according to claim 1 or 2 in the manufacture of medicaments.

6. Process for the preparation of compounds of the general formula I 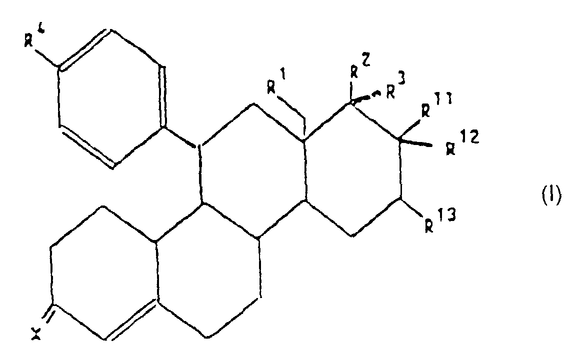 wherein X, R¹, R², R³, R⁴, R¹¹, R¹² and R¹³ have the meanings given in claim 1, characterised in that a compound of the general formula J or K 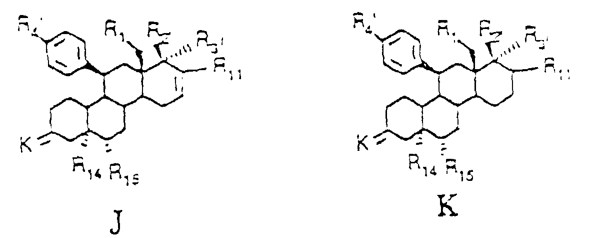 wherein
K is a keto group protected in the form of a ketal,
R¹ and R¹¹ have the same meanings as in formula I and R^{2'}, R^{3'} and R^{4'}, with the exception of the cyanide radical for R⁴, have the same meanings as R², R³ and R⁴, respectively, in formula I,
any hydroxy, mercapto, amino, oxo and/or terminal acetylene groups present being protected as necessary, and
R¹⁴ is a hydroxy group and
R¹⁵ is hydrogen
is subjected to the action of an acid agent that is capable of freeing the 3-oxy group and the other protected groups and is capable of splitting off water with the formation of the 4(5)-double bond, and then, if desired, any hydroxy, mercapto and/or amino group(s) present in R² and/or R³ and/or R⁴ is/are alkylated or acylated, if desired a cyanide radical is introduced into the 11β-aryl substituent, if desired the amino or sulphide group which may be present in R⁴ is oxidised, if desired reaction is carried out with hydroxylamine hydrochloride to form a product of the general formula I wherein X is the hydroxyimino grouping >N∼OH, or the 3-oxo group is converted into a product of the general formula I wherein X represents two hydrogen atoms, and optionally a pharmaceutically acceptable acid addition salt is prepared.

## Revendications

1. Composés répondant à la formule générale I 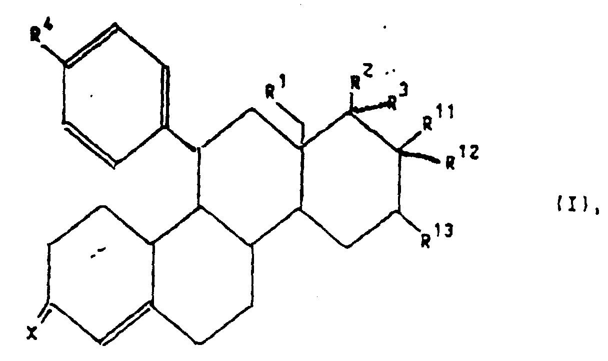 dans laquelle
X a la signification d'un atome d'oxygène, du groupement hydroxyimino >N∼OH ou de deux atomes d'hydrogène,
R¹ a la signification d'un atome d'hydrogène ou d'un groupe méthyle,
R² a la signification d'un groupe hydroxy, d'un groupe alcoxy en C₁-C₁₀ ou d'un groupe acyloxy en C₁-C₁₀,
R³ a la signification d'un atome d'hydrogène, du groupement -(CH₂)ₙCH₂Z, où n représente 0, 1, 2, 3, 4 ou 5, Z représente un atome d'hydrogène, le groupe cyano ou le radical -OR⁵, où R⁵ représente H, un groupe alkyle en C₁-C₁₀ ou un groupe acyle en C₁-C₁₀, du groupement -(CH₂)ₘC≡C-Y, où m représente 0, 1 ou 2 et Y représente un atome d'hydrogène, de fluor, de chlore, de brome ou d'iode, un radical hydroxyalkyle en C₁-C₁₀, alcoxyalkyle en C₁-C₁₀ ou acyloxyalkyle en C₁-C₁₀, ou, lorsque m est égal à 0, en supplément un groupe méthyle, du groupement -(CH₂)ₚ-CH=CH-(CH₂)ₖCH₂R⁶, où p représente 0 ou 1, k représente 0, 1 ou 2 et R⁶ représente un atome d'hydrogène, un groupe hydroxy, un radical alcoxy en C₁-C₄ ou un radical acyloxy en C₁-C₄, ou encore R² et R³ représentent conjointement un radical de la formule 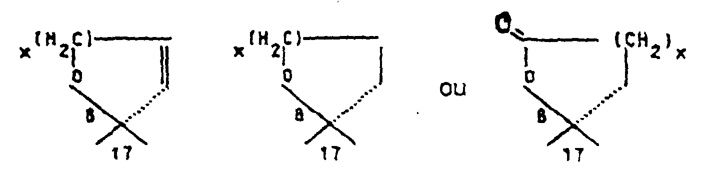 où x est égal à 1 ou à 2,
R⁴ a la signification d'un atome d'hydrogène, d'un groupe cyano, d'un atome de chlore, de fluor, de brome ou d'iode, d'un groupe trialkylsilyle ou trialkylstannyle, d'un radical alcoxyalkyle, acyle ou alkyle en C₁-C₈ linéaire ou ramifié, saturé ou insaturé, d'un groupe amino 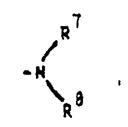 dans lequel R⁷ et R⁸ représentent indépendamment l'un de l'autre un atome d'hydrogène ou un groupe alkyle en C₁-C₄, ou d'un amino-oxyde correspondant 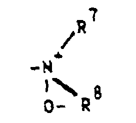 ou des groupements -OR⁹ ou -S(O)ᵢR⁹, où i est égal à 0, 1 ou 2 et R⁹ représente un atome d'hydrogène, un groupe méthyle, éthyle, propyle, isopropyle, méthoxyphényle, allyle ou 2-diméthylarninoéthyle, ou d'un radical hétéroaryle de la formule Iα 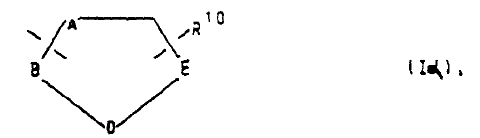 dans laquelle A représente un atome d'azote, d'oxygène ou de soufre, -B-D-E- représente la suite des éléments -C-C-C, -N-C-C- ou -C-N-C- et R¹⁰ représente un atome d'hydrogène, un groupe cyano, un atome de chlore, de fluor, de brome ou d'iode, un groupe trialkylsilyle ou trialkylstannyle, un radical alcoxyalkyle, acyle ou alkyle en C₁-C₈ linéaire ou ramifié, saturé ou insaturé, un groupe amino 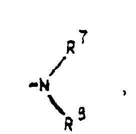 dans lequel R⁷ et R⁸ représentent indépendamment l'un de l'autre un atome d'hydrogène ou un groupe alkyle en C₁-C₄, ou un amino-oxyde correspondant 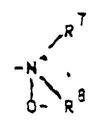 ou les groupements -OR⁹ ou -S(O)ᵢR⁹, où i est égal à 0, 1 ou 2, et R⁹ représente un atome d'hydrogène, un groupe méthyle, éthyle, propyle, isopropyle, méthoxyphényle, allyle ou 2-diméthylaminoéthyle,
ou d'un radical hétéroaryle de la formule Iβ 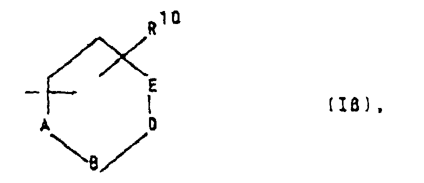 dans laquelle A représente un atome d'azote, et -B-D-E- représente la suite des éléments -C-C-C, -N-C-C-, -C-N-C- ou -C-C-N- et R¹⁰ a la signification déjà indiquée,
ou d'un radical phényle de la formule Iγ 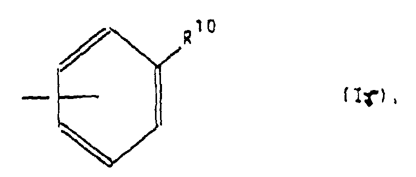 dans laquelle R¹⁰ a la signification déjà indiquée,
R¹¹ a la signification d'un atome de fluor, de chlore ou de brome,
R¹² et R¹³ formant alors conjointement une liaison supplémentaire, ou
R¹¹ a la signification d'un radical alkyle en C₁-C₄ linéaire ou ramifié ou d'un atome d'hydrogène, R¹² et R¹³ représentant alors chacun un atome d'hydrogène ou formant conjointement une liaison supplémentaire.

2. Composés suivant la revendication 1, à savoir
de la 17aβ-hydroxy-11β-(4-méthoxyphényl)-17aα-(1-propynyl)-17a-homooestra-4,16-dién-3-one,
de la 17aβ-hydroxy-11β-(4-méthoxyphényl)-17aα-méthyl-17a-homooestra-4,16-dién-3-one,
de la 11β-[4-(3-furannyl)phényl]-17aβ-hydroxy-17aα-(1-propynyl)-17a-homooestra-4,16-dién-3-one,
de la 11β-(4-acétylphényl)-17aβ-hydroxy-17aα-(1-propynyl)-17a-homooestra-4,16-dién-3-one,
de la 17aβ-hydroxy-17aα-(3-hydroxypropyl)-11β-[4-(3-pyridinyl)phényl]-17a-homooestra-4,16-dién-3-one,
du (Z)-4'-[17aβ-hydroxy-17aα-(3-hydroxy-1-propényl)-3-oxo-17a-homo-4,16-dién-11β-yl]-[1,1'-biphényl]-4-carbonitrile,
de la 11β-[4-(3-furannyl)phenyl)-17aβ-hydroxy-17aα-méthyl-17a-homooestra-4,16-dién-3-one,
de la 11β-(4-acétylphényl)-17aβ-hydroxy-17aα-(3-hydroxypropyl)-17a-homooestra-4-én-3-one,
de la 11β-(4-acétylphényl)-4',5'-dihydrospiro[17a-homooestra-4-ène-17aβ,2'(3H)-furann]-3-one,
du (Z)-4'-[17aβ-hydroxy-17aα-(3-hydroxy-1-propényl)-3-oxo-17a-homooestr-4-én-11β-yl]-[1,1'-biphényl]-4-carbonitrile,
de la 17-chloro-11β-(4-méthoxyphényl)-17aβ-hydroxy-17aα-(1-propynyl)-17a-homooestra-4,16-dién-3-one,
de la 17-chloro-17aβ-hydroxy-17aβ-(1-propynyl)-11β-[4-(3-pyridinyl)-phényl]-17a-homooestra-4,16-dién-3-one,
de la 11β-(4-acétylphényl)-17-chloro-17aβ-hydroxy-17aα-(1-propynyl)-17a-homooestra-4,16-dién-3-one,
de la 17-chloro-11β-[4-(3-furannyl)phényl]-17aβ-hydroxy-17aα-(1-propynyl)-17a-homooestra-4,16-dién-3-one,
de la 17-chloro-11β-[4-(3-furannyl)phényl]-17aβ-hydroxy-17aα-méthyl-17a-homooestra-4,16-dién-3-one,
du 4'-[17-chloro-17aβ-hydroxy-17aα-méthyl-3-oxo-17a-homooestra-4,16-dién-11β-yl][1,1'-biphényl]4-carbonitrile,
de la (Z)-11β-(4-acétylphényl)-17-chloro-17aβ-hydroxy-17aα-(3-hydroxy-1-propényl)-17a-tromooestra-4,16-dién-3-one,
de la 11β-(4-acétylphényl)-17-chloro-17aβ-hydroxy-17aα-(3-hydroxypropyl)-17a-homooestra-4,16-dién-3-one,
du 17-chlor-11β-[4-(3-furannyl)phényl]-17aβ-hydroxy-3-oxo-17a-homooestra-4,16-dién-17aα-acétonitrile,
de la 11β-(4-acétylphényl)-17-fluoro-17aβ-hydroxy-17aα-méthyl-17a-homooestra-4,16-dién-3-one,
de la 11β-(acétylphényl)-17-fluoro-17aβ-hydroxy-17aα-(3-hydroxypropyl)-17a-homooestra-4,16-dién-3-one,
de la 11β-(4-acétylphényl)-17-fluoro-17aβ-hydroxy-17aα-(1-propynyl)-17a-homooestra-4,16-dién-3-one,
de la 17aβ-hydroxy-11β-(4-méthoxyphényl)-17aα-(1-propynyl)-17a-homooestr-4-én-3-one.

3. Produits intermédiaires répondant à la formule générale F 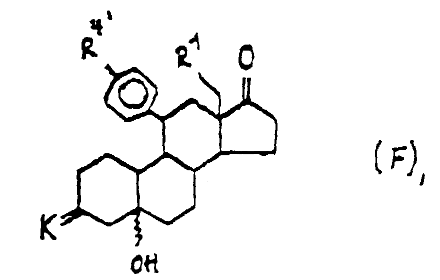 dans laquelle
K représente le groupe 1,2-éthanediylbis(oxy), 1,3-propanediylbis(oxy) ou 1,3-propane-(2,2-diméthyl)-diylbis(oxy) ou un autre oxygène-cétal,
R¹ représente un atome d'hydrogène ou un groupe méthyle, et
R^{4'} représente un des radicaux R⁴ déjà indiqués dans la revendication 1.

4. Préparations pharmaceutiques, contenant au moins un composé suivant l'une des revendications 1 et 2, ainsi qu'un support pharmaceutique.

5. Utilisation des composés suivant l'une des revendications 1 et 2, pour la fabrication de médicaments.

6. Procédé de préparation de composés répondant à la formule générale 1 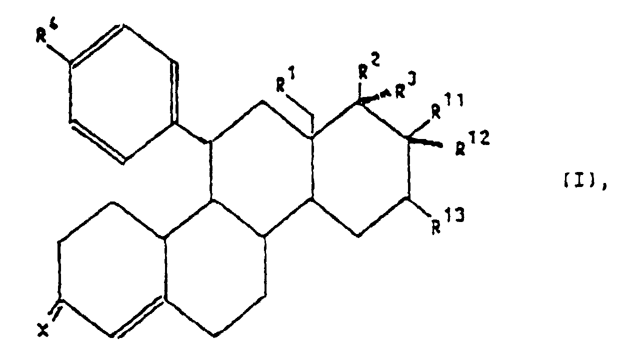 dans laquelle X, R¹, R², R³, R⁴, R¹¹, R¹² et R¹³ ont la signification indiquée dans la revendication 1, caractérisé en ce qu'un composé de la formule générale J ou K 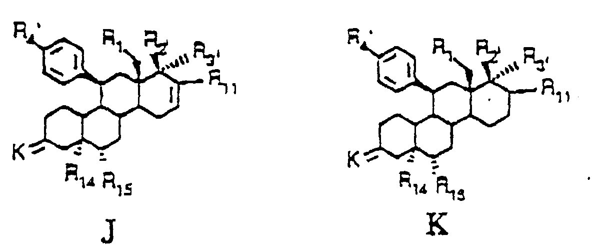 dans lesquels K représente un groupe cétonique protégé sous la forme de cétal, R¹ et R¹¹ ont la même signification que celle donnée dans la formule I, ainsi que R^{2'}, R^{3'} et R^{4'} ont, à l'exclusion du radical cyanure pour R⁴, la même signification que R², R³ et respectivement R⁴ dans la formule 1, des groupes hydroxy, mercapto, amino, oxo et/ou acétylène terminaux présents étant éventuellement protégés, R¹⁴ représentant un groupe hydroxy et R¹⁵ un atome d'hydrogène,
est soumis à l'action d'un agent acide qui est capable de libérer le groupe 3-oxy ainsi que les autres groupes protégés et d'éliminer de l'eau avec réalisation de la double liaison 4(5) et en ce qu'éventuellement ensuite un ou des groupes hydroxy, mercapto et/ou amino présents dans R² et/ou R³ et/ou R⁴ sont alkylés ou acylés, en ce qu'éventuellement un radical cyanure est introduit dans les substituants 11β-aryle, en ce que le groupe amino ou sulfure éventuellement contenu dans R⁴ est éventuellement oxydé, en ce qu'il est éventuellement transformé avec du chlorhydrate d'hydroxylamine en le produit de la formule générale I où X a la signification du groupement hydroxyimino >N∼OH ou en ce que le groupe 3-oxo est transformé en un produit de la formule générale I, où X représente deux atomes d'hydrogène, et en ce qu'éventuellement un sel d'addition d'acide pharmaceutiquement compatible est préparé.
